# EUROPEAN PATENT APPLICATION

(11) **EP 3 699 288 A1**
(43) Date of publication of application: **26.08.2020**
(21) Application number: 19216087.7
(22) Date of filing: 07.08.2015
(51) Int. Cl.: C12P 19/34, C12N 15/10, C12N 15/67

(54) **PROCESS FOR THE IN VIVO PRODUCTION OF RNA IN A HOST CELL**

(62) Divisional of application: 15760098.2
(71) Applicant: CureVac AG, 72076 Tübingen (DE)
(72) Inventor: SCHMID, Andreas, 72076 Tübingen (DE); STROBEL, Isabel, 72076 Tübingen (DE); EBER, Fabian Johannes, 72076 Tübingen (DE)
(74) Representative: Maiwald Patent- und Rechtsanwaltsgesellschaft mbH

(57) **Abstract**

The invention relates to a process for the *in vivo* production of a target RNA in a host cell comprising providing a microorganism comprising a vector comprising a DNA sequence encoding the target RNA, fermenting the host cell and allowing the DNA sequence to be transcribed into the target RNA, and obtaining the target RNA from the host cell. The invention also relates to the vector used in such process and the host cell comprising such vector as well as the use of the vector and the host cell in such process.

## Description

### Field of the Invention

The present invention is directed to processes for the *in vivo* production of a target RNA in host cells. The present invention is also directed to vectors comprising a DNA sequence encoding a target RNA and a DNA sequence encoding an RNase inhibitor, and host cells comprising the vectors. The present invention is further directed to the use of host cells and vectors for the *in vivo* transcription of a DNA sequence in processes for the production of the target RNA.

### Background of the Invention

The human genome comprises about 40.000 genes encoded by DNA. This genetic information is transcribed in RNA and then expressed into functional proteins.

Recombinant DNA as well as recombinant RNA have found use for the therapeutic administration in the context of immunotherapy, gene therapy or genetic vaccination.

Immunotherapy, gene therapy and genetic vaccination belong to the most promising and quickly developing methods of modern medicine. They may provide highly specific and individual treatment options for therapy of a large variety of diseases. Particularly, inherited genetic diseases, but also autoimmune diseases, infectious diseases, cancerous or tumor-related diseases as well as inflammatory diseases may be the subject of such treatment approaches. It is also envisaged to prevent (early) onset of such diseases by these approaches.

While DNA is known to be relatively stable and easy to handle, the use of DNA in therapy bears the risk of undesired insertion of the administered DNA fragments into the patient's genome potentially resulting in loss of function of the genes. As a further risk, the undesired generation of anti-DNA antibodies has emerged. Another drawback is the limited expression level of the encoded peptide or protein that can be achieved by DNA administration and its subsequent transcription/translation.

By using RNA instead of DNA for gene therapy or genetic vaccination, the risk of undesired genomic integration and generation of anti-DNA antibodies is minimized or can be even avoided. For many years it was generally accepted that mRNA is too unstable to be efficiently used for gene therapy purposes. In the last decade, however, several research groups faced this challenge and not only proved the feasibility of mRNA-mediated transfection with surprising results regarding transfection efficiency and duration of protein expression, but were also able to demonstrate major advantages over the use of DNA. One of these advantages is that mRNA does not need to cross the nuclear barrier for its encoded proteins to be expressed (reviewed in Tavernier et al., J Control Release. (2011); 150(3):238-47.

In order to further develop effective treatment options in the field of gene therapy and genetic vaccination, straight-forward processes for the recombinant production of RNA molecules in preparative amounts are highly desired. In the past only *in vitro* production of RNA was available to obtain preparative amounts which production requires the use of expensive recombinantly produced enzymes (e.g. RNA polymerases).

Only a few approaches for the *in vivo* production of RNA in preparative amounts have been established in the prior art.

In US 8,143,061 B2, an approach has been demonstrated based on the recombinant production and isolation of RNA from yeast mitochondria. This process includes the cloning of the recombinant RNA target sequence in a vector followed by transformation of the vector into yeast. The cytosolic RNA of the transformed yeast is degraded and the recombinant target RNA which was formed in the yeast mitochondria can be purified.

WO 02/24904 A2 discloses the production of RNA in microorganisms in which the RNA formed after transcription is to be secreted into the extracellular medium. However, the general feasibility of this approach is questionable, since the vector construct used in this method lacked a terminator signal and the ability of microorganisms to secrete large amounts of recombinant RNA is also doubtful.

In Nelissen et al. (2012) Nucl. Acids Res. 1-12 a process for producing a recombinant RNA molecule in *E.coli* using a tRNA-scaffold is shown.

In view of the above difficulties to create large amounts of RNA in a straight-forward manner, new processes allowing faster and cheaper production of preparative amounts of RNA are highly desired.

### Summary of the Invention

The present invention is directed to a process for producing a target RNA in a host cell comprising the steps of:
a) providing a host cell comprising a vector comprising a DNA sequence encoding the target RNA,
b) fermenting the host cell and allowing the DNA sequence to be transcribed into the target RNA,
c) obtaining the target RNA from the host cell.

Preferably, the host cell is a bacterial cell and more preferably it is *E.coli.*

Also preferably, the target RNA is selected from non-modified RNA and modified RNA, wherein the modified RNA comprises at least one modified nucleotide.

Preferably, the target RNA is selected from the group consisting of mRNA, viral RNA, retroviral RNA and replicon RNA, bicistronic or multicistronic RNA, small interfering RNA (siRNA), antisense RNA, CRISPR RNA, ribozymes, aptamers, riboswitches, immunostimulating RNA, ribosomal RNA (rRNA), small nuclear RNA (snRNA), small nucleolar RNA (snoRNA), microRNA (miRNA), and Piwi-interacting RNA (piRNA). Also preferably, the target RNA does not comprise a secondary structure of transfer RNA (tRNA).

Also preferably, the target RNA has a length of at least 100 or 200 nucleotides, more preferably of at least 300 or 400 nucleotides, even more preferably of at least 500 or 600 nucleotides and most preferably of at least 700 or 800 nucleotides. The target RNA has a length of not more than 10,000 nucleotides.

Preferably, the target RNA is an mRNA and codes for at least one antigen, a therapeutic protein, an antibody, a B cell receptor, a T cell receptor, or a fragment, variant or derivative thereof.

More preferably, the antigen is a tumor antigen, a pathogenic antigen, an allergenic antigen, or an autoimmune antigen.

Preferably, the host cell additionally comprises a DNA sequence encoding an RNase inhibitor. The DNA sequence encoding an RNase inhibitor can be located on the same vector as the DNA encoding the target RNA or it can be located on a second vector. The DNA sequence encoding an RNase inhibitor may also be integrated into the genome of said host cell.

In a preferred embodiment of the present invention the RNase inhibitor is selected from *E.coli* RNase inhibitor RraA and human RNase inhibitor RNH1.

Preferably, a compound capable of inhibiting peptide and/or protein synthesis is added in step b). More preferably, the compound capable of inhibiting peptide and/or protein synthesis is chloramphenicol.

In a preferred embodiment of the present invention a substance capable of inhibiting endogenous RNA polymerase is added in step b). More preferably, the substance capable of inhibiting endogenous RNA polymerase added in step b) is a rifamycin which is even more preferred rifampicin.

In a preferred embodiment of the present invention the target RNA is obtained from the host cell by separating the target RNA from the endogenous RNA of the host cell.

In this context it is particularly preferred that the step of obtaining the target RNA comprises a step of depleting the ribosomal RNA of the host cell and more preferably the ribosomal RNA of the host cell is depleted by capture hybridization of the ribosomal RNA with complementary oligonucleotides immobilized on a solid phase.

In a preferred embodiment of the present invention the target RNA is obtained by hybridization with a complementary nucleic acid sequence.

In a specific embodiment of the present invention the complementary nucleic acid sequence is immobilized on a solid matrix.

In another preferred embodiment of the present invention the target RNA comprises an affinity tag capable of binding to an affinity matrix.

Particularly preferably, the affinity tag comprises an aptamer, more preferably the aptamer is capable of binding to a structure useful for purification and even more preferably, the aptamer is capable of binding to Sephadex.

The affinity tag may also comprise an RNA nucleotide sequence capable of binding to a protein and/or peptide such asa boxB RNA binding peptide.

The target RNA may further comprise a ribozyme sequence, preferably an activatable ribozyme sequence, which is capable of cleaving the target RNA to delete the affinity tag. The ribozyme sequence is preferably located between the RNA to be produced and the affinity tag.

Preferably, the target RNA is obtained by binding of the affinity tag to an affinity matrix.

Also preferably, the affinity tag is cleaved from the target RNA by activating the activatable ribozyme sequence.

The present invention is further directed to a vector comprising a DNA sequence encoding a target RNA and a DNA sequence encoding an RNase inhibitor.

Preferably, the RNase inhibitor is selected from *E.coli* RNase inhibitor RraA and human RNase inhibitor RNH1.

Preferably, the target RNA comprises an affinity tag which is more preferably an aptamer and even more preferably an aptamer is capable of binding to Sephadex.

Also preferably, the target RNA further comprises a ribozyme sequence.

Also preferably, the DNA sequence encoding the target RNA is under the control of a promoter selected from the group consisting of T3 promoter, T7 promoter, SP6 promoter and tac promoter.

Also preferably, the vector further comprises a terminator selected from the T7 terminator, the VSV terminator, the PTH terminator, the *rrnB* T1 downstream terminator, the *rrnC* terminator, the concatemer junction sequence of the replicating T7 DNA,the rrnBT1T2 terminator and a variant of any of the foregoing.

The present invention is further directed to a host cell comprising the vector comprising a DNA sequence encoding a target RNA and a DNA sequence encoding an RNase inhibitor as further defined above.

In another preferred embodiment of the present invention, the host cell comprises a first vector comprising a DNA sequence encoding a target RNA and a second vector comprising a DNA sequence encoding an RNase inhibitor.

The RNase inhibitor may be selected from *E.coli* RNase inhibitor RraA and human RNase inhibitor RNH1.

Preferably, the host cell is a bacterium, more preferably it is *E.coli.*

In a preferred embodiment of the present invention the host cell, preferably *E.coli,* is capable of expressing a polymerase which may beselected from T3 polymerase, T7 polymerase and SP6 polymerase. Preferably, the expression of the polymerase is controlled by an operator such as the lac operator.

The *E.coli* may be selected from the group consisting of DH5α, BL21, JM109, HMS174, B834, SCS 110, XL1 Blue and XL10 Gold and preferably it isis selected from the group consisting of BL21(DE3), JM109(DE3), HMS174(DE3) and B834(DE3).

The present invention is further directed to the use of a host cell as defined above for the *in vivo* transcription of a DNA sequence encoding a target RNA into said target RNA.

The present invention is further directed to the use of a vector comprising a DNA sequence encoding an RNase inhibitor in a process for the *in vivo* transcription of a DNA sequence encoding a target RNA.

The RNase inhibitor may be selected from *E.coli* RNase inhibitor RraA and human RNase inhibitor RNH1.

### Brief Description of the Drawings

Figure 1 depicts the detection of the target RNA from vector construct IB-5 using RT-PCR following *in vivo* transcription in *E.coli.*
Figure 2 depicts the amounts of target RNA relative to the total amount of RNA isolated from the *in vivo* transcription with vector construct IB-1 (a), IB-3(b), IB-4 (c), IB-5 (d) and IB-6 (e) upon induction with different concentrations of IPTG.
Figure 3 depicts the chip electropherogram analysis of the target RNA obtained after *in vivo* transcription with vector construct IB-5 in *E.coli.*
Figures 4 and 5 depict alternative chip electropherogram analysis of the target RNA obtained after *in vivo* transcription with vector construct IB-5 in *E.coli.*
Figure 6 depicts northern blot analysis of the target RNA obtained after *in vivo* transcription with vector constructs IB-5 and IB-6 in *E.coli.*
Figure 7 depicts dot blot analysis with the target RNA obtained after *in vivo* transcription with vector constructs IB-1 and IB-3 to IB-6 in *E.coli.*

### Detailed Description of the Invention

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and nucleic acid chemistry and hybridization are those well known and commonly employed in the art. Standard techniques are used for nucleic acid and peptide synthesis. The techniques and procedures are generally performed according to conventional methods in the art and various general references (e.g., Sambrook et al., 1989, Molecular Cloning: A Laboratory Manual, 2d ed. Cold Spring Harbor Laboratory Press, Cold Spring Harbor, NY), which are provided throughout this document.

The process according to the present invention is an *in vivo* transcription process of a DNA sequence encoding a target RNA.

The process for producing a target RNA in a host cell comprises the steps of:
a) providing a host cell comprising a vector comprising a DNA sequence encoding the target RNA,
b) fermenting the host cell and allowing the DNA sequence to be transcribed into the target RNA,
c) obtaining the target RNA from the host cell.

The host cell in which the target RNA is produced by *in vivo* transcription of the DNA sequence encoding the target RNA may be any host cell which can be genetically modified to produce the target RNA and includes both prokaryotic and eukaryotic cells such as fungal cells, plant cells and animal cells. Preferably the host cell is a microorganism.

A microorganism is a microscopic living organism, which may be single celled or multicellular. Microorganisms are very diverse and include all the bacteria and archaea and almost all the protozoa. They also include some fungi, algae, and certain animals, such as rotifers.

The most suitable microorganism or host cell to be used in the process according to the present invention is a bacterial cell or strain. Suitable bacterial strains include for instance *Escherichia coli* (*E.coli*), *Corynebacterium, Lactococcus lactis,* Streptomyces, *Bacillus subtilis* and *Pseudomonas fluorescens.* However, many additional bacterial strains are known to be suitable for use in recombinant technology.

The most preferred bacterial strain to be used in the present invention is an *E.coli* strain.

Preferred *E.coli* strains to be used in the present invention include strains like DH5α, BL21, JM109, HMS174, B834, SCS110, XL1 Blue and XL10 Gold which are commercially available. Preferred microorganisms are those that are able to additionally express a recombinant polymerase, like bacterial strains with recombinant RNA polymerase activity. For instance, bacterial strains, like *E.coli* strains, are preferred that are able to express a recombinant polymerase selected from T3 polymerase, T7 polymerase and SP6 polymerase. Even more preferred *E.coli* strains are the DE3-modified strains of the strains mentioned above, derived from lambda phage DE3 carrying the T7 RNA polymerase gene under the control of the lac operator, such as BL21(DE3), JM109(DE3), HMS174(DE3) and B834(DE3).

Alternatively, common yeast expression systems like *Saccharomyces cerevisiae* and the methylotrophic yeast *Pichia pastoris* can also be used by simple adaptation of the process of the present invention to achieve *in vivo* RNA transcription in the corresponding host cell.

Other possible microorganisms within the meaning of the present invention include fungal-based systems, particularly filamentous fungi like those commonly used in recombinant expression of proteins, like *Aspergillus spp., Trichoderma reesei,* and *Neurospora crassa.*

It is also possible to use as host cells eukaryotic cell lines like human embryonal kidney (HEK) cells or Chinese hamster ovary (CHO) cells, or insect cells like *Spodoptera frugiperda* Sf9, Sf21 or High Five insect cells that are commonly used in recombinant expression of peptides or proteins.

Also plant cells can be used as host cells in the present invention to produce the target RNA by *in vivo* transcription of the DNA sequence encoding said target RNA.

The DNA sequence encoding the target RNA to be produced by the process of the present invention is cloned into an appropriate vector system that allows the transcription of the DNA sequence encoding the target RNA in a host cell, preferably a bacterial system after transfection/transformation.

Typical vectors to be used in the present invention are those that are commonly used in standard cloning and transcription procedures in the corresponding host cell. The term "vector" refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector, which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'-UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector, which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

A recombinant vector of the present invention can be constructed by standard techniques known to one of ordinary skill in the art and found, for example, in Sambrook et al. (1989) in Molecular Cloning: A Laboratory Manual. A variety of strategies are available for ligating fragments of DNA, the choice of which depends on the nature of the termini of the DNA fragments, and can be readily determined by persons skilled in the art.

The vectors may also contain other sequence elements to facilitate vector propagation and selection in bacteria and host cells. Coding sequences for selectable markers and reporter genes are well known to the person skilled in the art. In addition, the vectors of the present invention may comprise a recognition sequence for one or more restriction endonucleases.

Suitable vectors for cloning the target RNA to be produced according to the process of the present invention must include appropriate promoter and terminator regions as well as insertion/cloning sites for the DNA sequence encoding the target RNA, and optionally, for the DNA sequence encoding an RNase inhibitor. Suitable vectors also need to include a selectable marker gene that allows the straight-forward identification of positively transformed clones.

Preferred promoter regions in the vector are selected from the group consisting of T3 promoter, T7 promoter, T71ac promoter, SP promoter and tac promoter.

Preferred terminator regions in the vector are selected from T7 terminator, the VSV terminator, the PTH terminator, the *rrnB* T1 downstream terminator, the *rrnC* terminator, the concatemer junction sequence of the replicating T7 DNA, the rrnBT1T2 terminator and a variant of any of the foregoing.

The vector may further contain operator sequences to which a regulator protein binds and influences the affinity of the RNA polymerase to the promoter and therefore transcription. One particularly relevant example of an operator is the lac operator which is regulated by lactose or a synthetic lactose analogon such as IPTG. Preferred cloning vectors include the plasmids from the pUC or the pET(+) series, particularly the pET21(+) series (Novagen), pET-Duet-1 (Novagen), or pUCminusMCS (Blue Heron). When it is desired that the optional RNase inhibitor is co-expressed *in vivo,* one preferred vector is pET-Duet-1 (Novagen). In addition, the following plasmids can also be used: pT7Ts (GeneBank Accession No. U26404), vectors from the pGEM®series, for example pGEM®-1 (GeneBank Accession No. X65300) and pSP64 (GeneBank-Accession No. X65327).

Preferably, in the process of the present invention the *in vivo* transcription of the DNA sequence encoding the target RNA occurs without subsequently translating the target RNA, e.g. the formed messenger target RNA, into the protein that is optionally encoded by the target RNA.

If the host cell is a bacterial cell, the formation of the protein from the target RNA transcript after *in vivo* transcription can be preferably prevented by partially or completely deleting or mutating the Shine-Dalgarno sequence in the vector used for transcribing the RNA so that the formed target RNA will not be translated in the bacterial cell *in vivo* due to the absence of a functional ribosomal binding site that is required to bind the ribosome and initiate translation of the RNA to the encoded protein. In bacteria, the Shine-Dalgarno sequence is AGGAGG, and in *E.coli,* the sequence is known to be AGGAGGU. Nevertheless, the vector used for transcribing the RNA may include a Kozak sequence which is necessary for ribosome binding in eukaryotic cells, in particular if the target RNA is an mRNA which is to be translated in eukaryotic cells.In still a further embodiment the cells are treated with an antibiotic which inhibits translation of the RNA into protein. Antibiotics inhibiting the translation are known to the skilled person and include chloramphenicol, tetracycline, quinupristin/dalfopristin, fusidic acid, clindamycin and puromycin. Preferably, the antibiotic which inhibits translation of the RNA into protein is different from the antibiotic used for selecting stably transformed cells. Also preferably, the concentration of the antibiotic is such that it inhibits the cell's anabolic pathways, but does not cause the cells to die.

Additionally or alternatively, for further stabilizing the target RNA and maximizing the amount of target RNA formed in the process for *in vivo* transcription of a target RNA, a compound capable of inhibiting the endogenous RNA polymerase of the microorganism but not the RNA polymerase transcribing the target RNA from the vector, e.g. the T7 polymerase, may be added. One preferred compound is a rifamycin-type compound such as rifampicin, rifabutin and rifapentine. A particularly preferred rifamycin-type compound is rifampicin.

In one embodiment of the present invention, an additional DNA sequence encoding at least one RNase inhibitor is cloned into the vector comprising the DNA sequence encoding the target RNA.

An "RNase inhibitor" within the meaning of the present invention is a protein which binds to an RNase, thereby inibiting endonucleolytic cleavage of RNA catalyzed by said RNase.

The cloning strategy for the DNA sequence encoding the RNase inhibitor must be designed to allow the *in vivo* expression of a functional RNase inhibitor in the host cell in which the target RNA transcript is formed.

Alternatively, the expression of a functional RNase inhibitor in the microorganism in which the target RNA transcript is to be formed is achieved by providing the microorganism with a second vector comprising a DNA sequence encoding an RNase inhibitor.

In still another embodiment the DNA sequence encoding an RNase inhibitor may be integrated into the genome of the host cell.

Many RNase inhibitors are known in the art and in the present invention every possible RNase inhibitor can be used, preferably those that are capable of inhibiting the endogenous RNases of the microorganism used for the *in vivo* transcription of the target RNA, in particular *E.coli* RNase inhibitors. Suitable RNase inhibitors are selected from the group consisting of *E.coli* RNase inhibitors RRaA, RraB and YmdB and human RNase inhibitor RNH1 and combinations thereof. The *E.coli* RNase inhibitors RRaA and RraB are known to inhibit Ribonuclease E (RNase E) of *E.coli* (Lee et al. (2003) Cell 114(5): 623-634; Gao et al. (2006) Mol. Microbiol. 61(2): 394-406). The *E.coli* RNase inhibitor YmdB inhibits RNase III (Kim et al. (2008) Genes Dev. 22: 3497-3508) Most preferred is human RNase inhibitor RNH1. The nucleotide sequence of RRaA is available under Uniprot Accession Number POA8R0 and the nucleotide sequence of RNH1 is available under Uniprot Accession Number P13489.

The vectors of the present invention are introduced into the microorganism by conventional transformation or transfection techniques. The terms "transformation" and "transfection" refer to techniques for introducing foreign nucleic acid into the microorganism (or the host cell) and include calcium chloride transformation, lipofection and electroporation. Suitable methods for transforming or transfecting bacterial cells can for example be found in Sambrook et al. (Molecular Cloning: A Laboratory Manual, 2nd Edition, Cold Spring Harbor Laboratory press (1989)), and other laboratory manuals. Methods for introducing DNA into plant cells or mammalian cells *in vivo* are also well-known to the skilled person.

For transfection of the microorganism (or host cell), it is known that, depending upon the expression vector and transfection technique used, only a small fraction of cells may uptake the foreign DNA. In order to identify and select these transformants, a gene that encodes a selectable marker (such as resistance to antibiotics) may be introduced into the microorganism along with the DNA sequence encoding the target RNA and the DNA sequence encoding the RNase inhibitor. As used herein, the term "selectable marker" is used broadly to refer to markers which confer an identifiable trait to the indicator cell. Non-limiting examples of selectable markers include markers affecting viability, metabolism, proliferation, morphology and the like. Preferred selectable markers include those that confer resistance to drugs, such as ampicillin, kanamycin, tetracycline and chloramphenicol. Nucleic acids encoding a selectable marker may be introduced into a host cell on the same vector as that encoding the target RNA or may be introduced on a separate vector. Cells transfected with the introduced nucleic acid may be identified by drug selection (cells that have incorporated the selectable marker gene will survive, while the other cells die).

In the process of the present invention the host cell transformed with the vector comprising the DNA encoding the target RNA is fermented to produce the target RNA. The term "fermenting" means that the host cell is incubated in a medium providing all compounds necessary for cell growth and RNA and protein production at suitable conditions, e.g. a suitable temperature such as 37°C.

In bacteria, preferably in *E. coli,* the transcription of the target RNA may be induced by the addition of an inducing compound, preferably isopropyl-β-D-1-thiogalactopyranoside (IPTG), if either the DNA encoding the target RNA itself or the DNA encoding the RNA polymerase is linked to a suitable operator such as the lac operator. Before induction the bacterial cell is subjected to a fermentation process in order to reach a suitable cell density of the bacterial cells that allows a maximum level of viability and production of the target RNA transcript.

The cell density at the time of induction is comparable to cell densities that are typically used in recombinant protein expression using *E.coli.* Accordingly, the cell culture density OD₆₀₀ (measured with a spectrophotometer) should be in the range of 0.4 to 0.8, preferably 0.5 to 0.7, and most preferably at about 0.6. Typically, the growth of the cell culture should be in the log-phase or exponential phase.

Regarding the preferred *in vivo* transcription process involving a bacterial system, the preferred substance for inducing the transcription is isopropyl-β-D-1-thiogalactopyranoside (IPTG) though it is possible to use alternative inducing substances.

The induction of transcription is preferably achieved by concentrations of IPTG which are typically used in protein expression by *E.coli* strains. Typical IPTG concentrations are in the range of 0.1 mM to 1.5 mM, preferably from 0.2 to 1.2 mM, and more preferably from 0.5 to 1.0 mM, based on the total cell culture. Preferred concentrations of IPTG include 0.4 mM, 0.5 mM, 0.6 mM, 0.8 mM and 1.0 mM

The transcription is preferably induced at room temperature, e.g. at a temperature of about 21°C. Alternatively, induction temperatures can be selected below room temperature, for instance below 21°C, or even below 19°C, or even below 17°C.

The production period, i.e. the period from inducing transcription until obtaining the target RNA, is preferably selected in the medium range in order to achieve an appropriate balance between the maximum level of transcription on the one hand and the decreasing stability of the formed target RNA with increasing temperatures on the other hand. Accordingly, a preferred production period until obtaining the target RNA is in the range of 2 to 24 hours, more preferably in the range of 3 to 12 hours, and even more preferably in the range of 4 to 6 hours.

The target RNA may be stabilized during the process of *in vivo* transcription in order to avoid its degradation until it is obtained in isolated form, e.g. by adding special reagents to the crude mixture to preserve and stabilize the target RNA transcripts formed *in vivo* in the host cell, preferably in the bacterial cell culture. It is also especially important to prevent degradation of the target RNA after production phase when the crude mixture is stored before work-up and before obtaining the target RNA by carrying out subsequent purification procedures.

Such reagents for preventing gene expression and stabilization of formed transcripts of the target RNA are commercially available, such as RNAprotect cell reagent or RNA protect Bacteria Reagent, both available from Qiagen.

The addition of the above compounds during fermentation for stabilizing the target RNA and maximizing the amount of target RNA formed in the *in vivo* transcription process of the target RNA can be started and ended at certain phases of fermentation in order to optimize the stabilizing effect of such treatment. The skilled person will be able to establish the best scheme of addition for maximum efficiency of such treatment based on routine testing.

After the production phase in which the recombinant target RNA is formed in the host cells by the claimed *in vivo* transcription process, the target RNA is obtained. Preferably, the target RNA is specifically obtained, meaning that after the process of the present invention the target RNA is essentially free from the endogenous RNA of the host cell used for producing the target RNA. "Essentially free" means that less than 5% or 4%, preferably less than 3% or 2%, more preferably less than 1.5% or 1% and most preferably less than 0.8% or 0.5% endogenous RNA of the microorganism used for producing the target RNA is present in the target RNA obtained by the process of the present invention.

In the process of the present invention the target RNA is obtained from the cytoplasm of the microorganism, since it is not secreted into the cell culture medium.

The basic steps of obtaining the target RNA include first separating of the host cells in which the target RNA has been formed by *in vivo* transcription from the cell culture medium, then washing the host cells to remove cell culture medium, followed by lysis of the host cells and further purifying the target RNA by separating it from cell debris, endogenous proteins of the host cell and endogenous DNA and RNA of the host cell until the isolated target RNA is obtained.

For isolating the target RNA formed in the process of the present invention, the host cells, preferably bacterial cells, are harvested by separating them from the cell culture medium. One preferred method for the separation of the host cells, preferably the bacterial cells, from the culture medium is centrifugation by which the cells are obtained as cell pellet which can be processed further.

After separation of the cells from the medium, the cells are lysed to provide a crude mixture comprising the target RNA. Cell lysis is preferably carried out by enzymatic lysis, mechanical lysis and/or chemical lysis. There are many efficient techniques available in the art to efficiently disrupt the host cells. These techniques are known to the skilled person. For instance, enzymatic lysis may be carried out by using lysozyme. Mechanical or physical lysis includes mechanical disruption, liquid homogenization, the application of high frequency sound waves, e.g. ultrasonification, the application of freeze/thaw cycles and manual grinding.

After lysis of the host cells and obtaining the crude mixture, the RNA is further purified. Typically, in a first step the total RNA of the host cell is separated from other molecules within the host cell and then in a second step the target RNA is specifically obtained.

Conventional methods for the isolation of RNA can be preferably applied like for instance the phenol/chloroform extraction and precipitation of RNA with which the skilled person will be familiar as one standard procedure in the field of recombinant RNA technology.

Additionally or alternatively, one of the various kit products which are commercially available for the isolation of RNA can be used. Such kit products are based on special solid matrices that are capable of binding RNA molecules with relatively high selectivity. One example for such kit is the RNeasy Mini Kit® (Qiagen).

Further purification methods useful for isolation of RNA are preferably selected from cation exchange chromatography, anion exchange chromatography, membrane absorbers, reversed phase chromatography, normal phase chromatography, size exclusion chromatography, hydrophobic interaction chromatography, mixed mode chromatography, affinity chromatography, hydroxylapatite (HA) chromatography, or combinations thereof.

Further purification of the target RNA from the crude mixture can be achieved based on alternative established methods, like for instance those disclosed in WO 2014/140211 A1 and the references cited therein.

The target RNA is further obtained from the microorganism by separating the target RNA from the endogenous RNA of the microorganism. Optionally, separating the target RNA from the endogenous RNA of the microorganism comprises depleting the ribosomal RNA from the crude mixture. The ribosomal RNA is preferably depleted by capture hybridization of the ribosomal RNA with oligonucleotides that have the ability to selectively bind ribosomal RNA, particularly bacterial 16 S and 23S ribosomal RNA and that are immobilized on a solid phase. One possibility is to deplete the ribosomal RNA in the crude mixture by the use of commercially available kits like the MICROBExpress™ kit (Ambion) which is based on a capture hybridization approach using magnetic beads.

Alternatively or additionally, the target RNA can be obtained from the crude mixture by affinity chromatography in which the target RNA is trapped by a solid or stationary phase or medium which has selective affinity to the target RNA. One preferred stationary phase is a Sephadex column carrying an immobilized ligand with affinity to the target RNA. Other molecules in the crude mixture will not be bound to the ligand, since these molecules lack the required affinity. The stationary phase with the target RNA bound to the ligand can then be removed from the crude mixture, washed and the target RNA is obtained again in enriched form after removing the target RNA from the affinity ligand. Depending on the strength of the interaction between ligand and target RNA, the target RNA can be removed by a change of salt concentration, ionic strength, pH value and the like. For instance, elution can be accomplished by application of a pH or salt gradient to the stationary phase.

The use of affinity chromatography to obtain the target RNA after *in vivo* transcription in the microorganism is preferred because the target RNA can be obtained in a straight-forward manner avoiding multiple consecutive purification steps that normally lead to considerable loss of product due to continued slow degradation and accumulation of product losses along a series of consecutive purification steps.

One preferred embodiment of an affinity purification scheme is based on immobilizing a nucleic acid ligand on the stationary phase or solid matrix which is capable of hybridizing with the target RNA via the conventional rules of base-pairing. The DNA ligand will have a nucleic acid sequence which is sufficiently complementary to bind the target RNA with a strength which is sufficient to maintain binding during the washing steps.

In another preferred purification method for obtaining the target RNA, the target RNA comprises an affinity tag which is capable of binding to an affinity matrix and which can be removed from the target RNA after purification. The affinity tag may be attached to the target RNA via a linker between the target RNA and the affinity tag sequence. The linker may comprise 10 nucleotides or less and may comprise any nucleotides or combinations thereof.

In one preferred embodiment the affinity tag comprises an additional RNA sequence which is capable of binding to a matrix e.g. Sephadex or to a protein and/or peptide with high selectivity. The affinity tag preferably comprises the additional RNA sequence upstream or downstream of the target RNA sequence.

In a particularly preferred embodiment the additional RNA sequence is an aptamer. Aptamers are short oligonucleotides usually comprising 25 to 70 nucleotides which are capable of binding to a specific molecule due to their specific 3D structure. Examples of suitable aptamers for use in the present invention are those that bind to a structure useful for purification such as Sephadex aptamer, glutathione aptamer, cellulose aptamer, Cibacron blue aptamer and streptavidin aptamer (Srisawat and Engelke (2001) RNA 7: 632-641) which are able to bind to common chromatography matrices. More preferably, the aptamer is capable of binding to Sephadex. Such an aptamer is described in Srisawat et al. (2001) Nucl. Acids Res. 29(2) e4 and has a core sequence of GAGUAAUUUACG (SEQ ID No. 9).

In one particularly preferred embodiment, the target RNA further comprises an activatable ribozyme sequence, wherein the activatable ribozyme sequence is preferably inserted between the DNA sequence encoding the target RNA and the DNA sequence encoding the additional RNA sequence in order to allow subsequent release of the target RNA from the additional RNA sequence used as affinity tag by triggering autocleavage of the activatable ribozyme sequence.

A ribozyme is a catalytic nucleic acid molecule which is an RNA molecule capable of catalyzing reactions including, but not limited to, site-specific cleavage of other nucleic acid molecules such as RNA molecules. The term ribozyme is used interchangeably with terms such as catalytic RNA, enzymatic RNA, or RNA enzyme.

In the early 80s natural RNA molecules were discovered which are capable of catalyzing reactions in the absence of any protein component and these molecules were called ribozymes. Several classes of ribozymes occurring in natural systems have been discovered, most of which catalyse intramolecular splicing or cleavage reactions (reactions '*in cis*'). Since most of the naturally occurring ribozymes catalyse self-splicing or self-cleavage reactions, it was necessary to convert them into RNA enzymes which can cleave or modify target RNAs without becoming altered themselves (reactions '*in trans*')*.*

Ribozymes are broadly grouped into two classes based on their size and reaction mechanisms: large and small ribozymes. The first group consists of the self-splicing group I and group II introns as well as the RNA component of RNase P, whereas the latter group includes the hammerhead, hairpin, hepatitis delta ribozymes and varkud satellite (VS) RNA as well as artificially selected nucleic acids. Large ribozymes consist of several hundreds up to 3000 nucleotides and they generate reaction products with a free 3'-hydroxyl and 5'-phosphate group. In contrast, small catalytically active nucleic acids from 30 to ∼150 nucleotides in length generate products with a 2'-3'-cyclic phosphate and a 5'-hydroxyl group (Schubert and Kurreck, 2004. Curr. Drug Targets 5(8):667-681).

Group I introns include the self-splicing intron in the pre-ribosomal RNA of the ciliate *Tetrahymena thermophilia.* Further examples of group I introns interrupt genes for rRNAs, tRNAs and mRNAs in a wide range of organelles and organisms. Group I introns perform a splicing reaction by a two-step transesterification mechanism: The reaction is initiated by a nucleophilic attack of the 3'-hydroxyl group of an exogenous guanosine cofactor on the 5'-splice site. Subsequently, the free 3'-hydroxyl of the upstream exon performs a second nucleophilic attack on the 3'-splice site to ligate both exons and release the intron. Substrate specificity of group I introns is achieved by an Internal Guide Sequence (IGS). The catalytically active site for the transesterification reaction resides in the intron, which can be re-engineered to catalyse reactions *in trans.*

Group II introns are found in bacteria and in organellar genes of eukaryotic cells. They catalyse a self-splicing reaction that is mechanistically distinct from group I introns because they do not require a guanosine cofactor. Instead, the 2'-hydroxyl of a specific adenosine at the so-called branch site of the intron initiates the reaction by a nucleophilic attack on the splice-site to form a lariat-type structure.

RNase P was the first example of a catalytic RNA that acts *in trans* on multiple substrates. RNase P can be considered to be the only true naturally occurring trans-cleaving RNA enzyme known to date. However, for full enzymatic activity under *in vivo* conditions the protein component is essential.

The hammerhead ribozyme is found in several plant virus satellite RNAs, viroids and transcripts of a nuclear satellite DNA of newt. This ribozyme is the smallest of the naturally occurring ribozymes and processes the linear concatamers that are generated during the rolling circle replication of circular RNA plant pathogens. The development of hammerhead variants that cleave target RNA molecules *in trans* was a major advancement that made possible the use of ribozyme technology for practical applications. The hammerhead ribozyme motif that has widely been applied since then comprises three helical sections connected via a three-way helical junction.

In hairpin ribozymes the catalytic entity is part of a four-helix junction. A minimal catalytic motif containing approximately 50 nucleotides has been identified that can be used for metal-ion dependent cleavage reactions *in trans.* It consists of two domains, each harbouring two helical regions separated by an internal loop, connected by a hinge region. One of these domains results from the association of 14 nucleotides of a substrate RNA with the ribozyme via base-pairing.

The hepatitis delta virus (HDV) ribozyme is found in a satellite virus of hepatitis B virus. Both the genomic and the antigenomic strand express cis-cleaving ribozymes of ∼85 nucleotides that differ in sequence but fold into similar secondary structures. The crystal structure of the ribozyme reveals five helical regions are organized by two pseudoknot structures. The catalytic mechanism of the hepatitis delta virus ribozyme appears to involve the action of a cytosine base within the catalytic centre as a general acid-base catalyst. The hepatitis delta ribozyme displays high resistance to denaturing agents like urea or formamide. Trans-cleaving derivatives of this ribozyme have been developed.

The Varkud Satellite (VS) ribozyme is a 154 nucleotide long and is transcribed from a plasmid discovered in the mitochondria of certain strains of Neurospora. The VS ribozyme is the largest of the known nucleolytic ribozymes

An activatable ribozyme may be activated by a variety of ways, including by effector molecules and/or other means such as radiation (e.g., light radiation, UV radiation, IR radiation), as well as temperature and/or pH changes. Preferably, the activatable ribozyme is activated by the addition of Mg2+.

Activatable ribozymes are well known in the art and include, for example, those described in Koizumi et al., (1999) Nature Struct. Biol. 6(11): 1062-1071 which are activatable by specific nucleoside 3',5'-cyclic monophosphate compounds such as cGMP or cAMP, those described in Grate and Wilson, (1999) Proc. Nat. Acad. Sci 96: 6131-6136 (a malachite green (MG)-tagged RNA that cleaves upon laser irradiation) as well as the Glucosamine-6-phosphate activated (GlmS) ribozyme which is activated by glucosamine-6-phosphate (GlcN6P). Other examples of suitable activatable ribozymes include virus-derived activatable ribozymes such as activatable hepatitis delta virus ribozyme (H5V) (Shih et al., Annual Review of Biochemistry 71: 887-917 (2002)). In a further preferred embodiment, the above-mentioned activatable ribozyme sequence is a sequence of a glmS ribozyme, which is typically produced by Gram-positive bacteria such as *Bacillus subtilis* and *Bacillus anthracis* (Roth, A. et al. (2006) RNA 12: 607-619). In a further embodiment, the above-mentioned glmS ribozyme is a *Bacillus anthracis* glmS ribozyme sequence.

Moreover, methods for developing ribozymes capable of being activated by specific effector molecules are well known (see for example, US Patent No. 6,630,306; Winkler et al., (2004) Nature 428: 281-286; Koisumi *et al.,* 1999, supra; and Seetherman et al., (2001) Nature Biotech. 19: 336-341). Notably, activatable ribozymes may be generated by combining a ribozyme and a riboswitch, as described in Chen and Elington, PLoS Comput Biol 2009 5(12): e1000620.

Alternatively, the additional RNA nucleotide sequence of the affinity tag is an bacteriophage boxB RNA sequence which is an RNA hairpin sequence comprising short stems of typically 5 to 7 base pairs with a 5 or 6 nucleotide loop as found in some bacteriophages, preferably those of the Lambda family of bacteriophages like lambda, φ21 and P22.

The "bacteriophage boxB RNA sequences" have a strong affinity for specific phage-derived polypeptides/peptides involved in the regulation of transcription (e.g., transcription termination, transcription anti-termination), such as bacteriophage N proteins and Coliphage HK022 nun protein, and more particularly for the amino-terminal region of such polypeptides/peptides.

Examples of bacteriophage boxB RNA sequences are described in Cilley and Williamson RNA (2003) 9: 663- 676; Neely and Friedman, Molecular Microbiology (2000) 38(5): 1074-1085 and Chattopadhyay et al. (1995) Proc. Natl. Acad. Sci. 92: 4061 -4065.

In one embodiment, the above-mentioned bacteriophage boxB RNA sequence is a bacteriophage lambda (λ) boxB RNA sequence. The above-mentioned bacteriophage boxB RNA sequence and/or activatable ribozyme sequence may be incorporated at the 5' or 3' end of the target RNA sequence, or within the target RNA sequence. In a preferred embodiment, the above-mentioned bacteriophage boxB sequence and/or activatable ribozyme sequence is/are incorporated at the 3' end of the target RNA sequence, preferably in a way that the activatable ribozyme sequence is inserted between the target RNA and the bacteriophage boxB sequence.

In another embodiment, the above-mentioned method further comprises incorporating a linker at the 3' end of said target RNA (e.g. between the 3' end of the target RNA and the affinity tag sequence). In an embodiment, the linker comprises 10 nucleotides or less and comprises any nucleotides or combinations thereof. In an embodiment, the linker comprises 5 nucleotides or less, and in a further embodiment it comprises 1 or 2 nucleotides.

The above-mentioned bacteriophage boxB sequence may be incorporated at the 5' end, at the 3' end or within the activatable ribozyme sequence. In a preferred embodiment, the above-mentioned bacteriophage boxB sequence is incorporated at the 3' end of the activatable ribozyme sequence. In another embodiment, the above-mentioned bacteriophage boxB sequence is incorporated into a stem-loop of the activatable ribozyme sequence, in a further embodiment into the variable apical P1 stem-loop of the activatable ribozyme sequence.

The affinity tag on the target RNA sequence comprising the activatable ribozyme sequence and a bacteriophage boxB RNA sequence as decribed above may be immobilized on the solid matrix by binding to a peptide/polypeptide construct comprising a) a boxB RNA binding peptide, b) an immobilizing moiety and, optionally, c) a peptide linker linking the boxB RNA binding peptide and the immobilizing moiety.

The term "boxB RNA binding peptide" as used herein, refers to phage-derived peptides capable of binding with high affinity to a bacteriophage boxB RNA sequence as defined above. Typically, these phage-derived peptides are derived from specific regions of proteins (generally arginine-rich motifs located in the N-terminal portion) involved in the regulation of transcription (e.g. termination), such as bacteriophage N proteins and coliphage HK022 Nun proteins.

As used herein, the term "bacteriophage N peptide" refers to an arginine-rich peptide motif (ARMs) derived from bacteriophage N proteins and having affinity for a bacteriophage boxB RNA. Examples of bacteriophage N peptides are disclosed in Cilley and Williamson, RNA (2003) 9: 663-676; Su et al., Biochemistry (1997), 36: 12722-12732 and Austin *et al.,* 2002, supra.

Bacteriophage N peptide as used herein also encompass derivatives of naturally occurring or mutated N peptides, for example acridine derivatives as described in Qi et al., Biochemistry (2010) 49: 5782-5789.

In a further embodiment, the above-mentioned boxB RNA binding peptide is a peptide derived from Coliphage HK022 Nun protein, and more particularly comprises residues 1 to 44, e.g. residues 10-44 or 20-44, of the Coliphage HK022 Nun protein (Faber et al. J. Biol. Chem. 276(34): 32064-32070).

In a further embodiment, the above-mentioned boxB RNA binding peptide (e.g. bacteriophage N peptide) binds to said bacteriophage boxB with a dissociation constant (K_{D}) of about 2 x 10⁻⁸ M or less at physiological salt concentrations (about 150 mM).

In further embodiments, the above-mentioned boxB RNA binding peptide (e.g. bacteriophage N peptide) binds to said bacteriophage boxB with a K_{D} of about 5 x 10⁻⁸ M or less, about 2 x 10⁻⁸ M or less, 1 x 10⁻⁸ M or less, about 5 x 10⁻⁹ M or less, about 1 x 10⁻⁹ M or less, or about 1 x 10⁻¹⁰ or less at physiological salt concentrations (about 150 mM).

In another embodiment, the above-mentioned boxB RNA binding peptide comprises from about 10 to about 40 amino acids, in further embodiments from about 10 to about 30, from about 13 to about 25, from about 15 to about 21 amino acids, from about 17 to about 20 amino acids.

In further embodiments, the above-mentioned peptide/polypeptide construct comprises a plurality of boxB RNA binding peptides (e.g. bacteriophage N peptide), either as multiple copies of the same peptide, or as different peptides. The plurality of boxB RNA binding peptides may be covalently linked either directly (e.g. through a peptide bond) or via a suitable linker moiety, e.g. a linker of one or more amino acids (e.g. a polyglycine linker or the like) or another type of chemical linker (e.g. a carbohydrate linker, a lipid linker, a fatty acid linker, a polyether linker, PEG, etc. (see, e.g. Hermanson (1996) Bioconjugate techniques). In a further preferred embodiment, the above-mentioned boxB RNA binding peptide (e.g. bacteriophage N peptide) comprises at its carboxy-terminal end a peptide linker that links the bacteriophage N peptide and the moiety capable of binding to the solid support (immobilizing or binding moiety).

Therefore, in one especially preferred embodiment, the configuration of the peptide/polypeptide construct (preferably from N- to C-terminus) binding the target RNA with the activatable ribozyme sequence and the additional RNA nucleotide sequence (being preferably the bacteriophage boxB RNA sequence) as decribed above is as follows:
boxB RNA binding peptide - peptide linker - immobilizing moiety

The boxB binding peptide has already been described above.

The peptide linker may be any amino acid sequence, such as a natural (e.g. a naturally occurring peptide or polypeptide) or an artificial (e.g. a synthetic, non-naturally occurring peptide or polypeptide) sequence, that does not significantly interfere with the binding of the boxB RNA binding peptide to the bacteriophage boxB RNA sequence) and that permits the binding of the immobilizing moiety to the solid support (e.g. that does not significantly interfere with the binding of the immobilizing moiety to the solid support). As noted above, the above-mentioned construct further comprises a moiety capable of binding to said solid support (an immobilizing or binding moiety). The immobilizing moiety is linked to the peptide linker and is thus indirectly fused (i.e. through the peptide linker) to the C- terminus of the boxB RNA binding peptide (e.g. bacteriophage N peptide). The immobilizing moiety may be any moiety capable of binding, either directly or indirectly, to a solid support. In an embodiment using the boxB RNA binding peptide, the solid support comprises a moiety or ligand capable of binding to the immobilizing moiety of the polypeptide construct.

Moieties capable of binding to a solid support useful for affinity purification are well known in the art. For example, a polyhistidine tag (commonly referred to as His-tag) is a moiety comprising a plurality of histidine residues (at least 5, typically 6) which are capable of binding a solid support that contains bound metal ions, and more particularly nickel or cobalt. Other known binding moieties useful for affinity purification include biotin-based tags (which binds to avidin, streptavidin or analogs/derivatives thereof), Strep tag (a short peptide of 8 amino acids) which binds to Sfrep-Tactin™ an engineered form of streptavidin (commercially available from Qiagen), as well as Glutathione S-transferase (GST) tag, which binds to a glutathione-containing solid support. Any affinity tag-based system may be used in the constructs and methods of the present invention.

The above described affinity purification procedure which is based on the high binding affinity of bacteriophage boxB RNA sequences to bacteriophage boxB RNA binding peptides is also disclosed in WO 2012/006732 A9 which is incorporated herein by reference.

In the method of the present invention the target RNA is preferably obtained by specifically binding the target RNA to a solid support via the affinity tag which may either comprise an aptamer or a bacteriophage boxB RNA sequence. The term "solid support" (or "solid matrix") generally refers to any material to which a biospecific ligand is covalently attached, such as chromatographic media (e.g. resin, gel, beads) that are generally used to purify molecules and macromolecules based on various properties (e.g. size, charge, affinity for a given ligand, etc.). Solid supports are well known in the art and include, for example, matrices of polyacrylamide resins or cross-linked polysaccharides such as cross-linked dextran (e.g. Sephadex™), cross-linked agarose (e.g. Sepharose™) and the like.

In further preferred embodiments, binding to the solid support (on which the above-mentioned construct is bound) may for example be achieved by batch treatment or column chromatography.

Batch treatment typically entails combining the sample (containing the target RNA of the present invention) with the solid support in a vessel to allow binding of the target RNA, mixing, separating the solid support (e.g. by centrifugation), removing the liquid phase, washing, separating the solid support (e.g., re- centrifuging), adding an elution buffer, separating the solid support (e.g. re-centrifuging) and removing the eluate.

Column chromatography typically entails packing the solid support onto a chromatography column, passing the sample (containing the target RNA of the present invention) through the column to allow binding of the RNA, passing a wash buffer through the column and subsequently an elution buffer to collect the bound material. Hybrid approaches may also be used, for example binding via a batch method followed by packing the solid support with the bound target RNA onto a column, followed by washing and elution on the column.

In further embodiments, the batch method can also be combined with the use of spin cups and Steriflip™ filter units, which typically improve resin recovery.

By the method of the present invention the target RNA is obtained in a purity of at least 95% or 96%, preferably of at least 97% or 98%, more preferably of at least 98.5% or 99% and most preferably of at least 99.2% or 99.5%. The purity is determined by calculating the percentage of the target RNA relative to the total RNA obtained by the process of the present invention.

The DNA sequence encoding the target RNA sequence to be produced by the present process of *in vivo* transcription refers to the sequence of nucleotides consisting of deoxyadenosine monophosphate, deoxythymidine monophosphate, deoxyguanosine monophosphate and deoxycytidine monophosphate monomers which are - by themselves - composed of a sugar moiety (deoxyribose), a base moiety and a phosphate moiety. These nucleotides polymerise to form a polynucleotide with a characteristic backbone structure. The backbone structure is, typically, formed by phosphodiester bonds between the sugar moiety of the nucleotide, i.e. deoxyribose, of a first and a phosphate moiety of a second, adjacent monomer. In the double stranded form, the nucleotides of the first strand typically hybridize with the nucleotides of the second strand, e.g. by AT-base-pairing and G/C-base-pairing. The specific order of the monomers in the DNA sequence, i.e. the order of the bases linked to the sugar/phosphate-backbone is called the DNA sequence.

With respect to the target RNA and its sequence of nucleotides, the DNA sequence encoding the target RNA refers to the sequence of DNA nucleotides which is complementary to the sequence of the target RNA obtained by transcription of this DNA sequence.

The term "RNA" is the usual abbreviation for ribonucleic acid. It is a nucleic acid molecule, i.e. a polymer consisting of nucleotides. These nucleotides are usually adenosine monophosphate, uridine monophosphate, guanosine monophosphate and cytidine monophosphate monomers, which are connected to each other along a so-called backbone. The backbone is formed by phosphodiester bonds between the sugar, i.e. ribose, of a first and a phosphate moiety of a second, adjacent monomer. The specific succession of the monomers is called the RNA sequence or sequence of RNA. Usually RNA is obtained by transcription of a DNA sequence, e.g., inside a cell. In eukaryotic cells, transcription is typically performed inside the nucleus or the mitochondria.

The sequence forming the target RNA according to the present invention refers to a RNA polynucleotide sequence which can comprise modified or non-modified (un-modified) nucleotides thereby forming modified or non-modified target RNA depending on the presence or absence of modified RNA nucleotides.

If at least one modified RNA nucleotide is present in the polynucleotide sequence of the target RNA according to the present invention the target RNA forms a modified target RNA, otherwise it is a non-modified or un-modified target RNA. The production of non-modified target RNA is preferred in the process of the present invention.

The term "non-modified RNA" or "non-modified target RNA" refers to a RNA sequence comprising nucleotides which are present in nature. For example, a non-modified reference mRNA corresponds to an mRNA sequence comprising naturally occurring nucleotides, including nucleotides comprising naturally occurring modifications, and/or naturally occurring untranslated regions, such as the naturally occurring 5'-CAP structure m7GpppN, optionally the naturally occurring 5'-UTR, if present in the naturally occurring mRNA sequence, the wild type open reading frame, optionally the naturally occurring 3'-UTR if present in the naturally occurring mRNA sequence and a poly A sequence with approximately 30 adenosines.

The term "modified RNA" comprises any type of ribonucleic acid molecule that is modified such that the amount of protein or peptide produced from said modified RNA measured at a specific time point or over a specific time period is increased in comparison with RNA lacking the modification ("unmodified reference RNA"). Methods for measuring increased protein expression of a modified RNA relative to non-modified RNA are for instance disclosed in WO 2015/024667 A1 which is herein incorporated by reference.

The term "RNA modification" as used herein may refer to chemical modifications comprising backbone modifications as well as sugar modifications, base modifications or lipd modifications.

In this context, the modified RNA molecule as defined herein may contain nucleotide analogues/modifications, e.g. backbone modifications, sugar modifications or base modifications. A backbone modification in connection with the present invention is a modification, in which phosphates of the backbone of the nucleotides contained in a nucleic acid molecule as defined herein are chemically modified. A sugar modification in connection with the present invention is a chemical modification of the sugar of the nucleotides of the nucleic acid molecule as defined herein. Furthermore, a base modification in connection with the present invention is a chemical modification of the base moiety of the nucleotides of the nucleic acid molecule of the nucleic acid molecule. In this context nucleotide analogues or modifications are preferably selected from nucleotide analogues which are applicable for transcription and/or translation.

### Sugar Modifications:

The modified nucleosides and nucleotides, which may be incorporated into the modified RNA as described herein, can be modified in the sugar moiety. For example, the 2' hydroxy! group (OH) can be modified or replaced with a number of different "oxy" or "deoxy" substituents. Examples of "oxy"-2' hydroxyl group modifications include, but are not limited to, alkoxy or aryloxy (-OR, e.g. R = H, alkyl, cycloalkyl, aryl, aralkyl, heteroaryl or sugar); polyethyleneglycols (PEG), -O(CH₂CH₂O)nCH₂CH₂OR; "locked" nucleic acids (LNA) in which the 2' hydroxyl is connected, e.g. by a methylene bridge, to the 4' carbon of the same ribose sugar; and amino groups (-O-amino, wherein the amino group, e.g. NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH₂; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified RNA can include nucleotides containing, for instance, arabinose as the sugar.

### Backbone Modifications:

The phosphate backbone may further be modified in the modified nucleosides and nucleotides, which may be incorporated into the modified RNA, as described herein. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

### Base Modifications:

The modified nucleosides and nucleotides, which may be incorporated into the modified RNA, as described herein, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group. In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl inosine-5'-triphosphate 4-thiouridine-5'-triphosphate, 5-aminoallylcytidine-5'-triphosphate, 5-aminoallyluridine-5'-triphosphate, 5-bromocytidine- 5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-lodo-2'-deoxycytidine-5'-triphosphate, 5-iodouridine-5'-triphosphate, 5-lodo-2'-deoxyuridine-5'-triphosphate, 5-methylcytidine-5'-triphosphate, 5-methyluridine-5'-triphosphate, 5- Propynyl-2'-deoxycytidine-5'-triphosphate, 5-Propynyl-2'-deoxyuridine-5'-triphosphate, 6- azacytidine-5'-triphosphate, 6-azauridine-5'-triphosphate, 6-chloropurineriboside-5'- triphosphate, 7-deazaadenosine-5'-triphosphate, 7-deazaguanosine-5'-triphosphate, 8- azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine- 5 '-triphosphate, or puromycin-5'-triphosphate, xanthosine-5'-triphosphate. Particular preference is given to nucleotides for base modifications selected from the group of base- modified nucleotides consisting of 5-methylcytidine-5'-triphosphate, 7-deazaguanosine-5'- triphosphate, 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl- pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio- uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza- pseudouridine, dihydrouridine, dihydropseudouridine, 2-thio-dihydrouridine, 2-thio-dihydropseudouridine, 2-methoxyuridine, 2-methoxy-4-thio-uridine/4-methoxy-pseudouridine, and 4-methoxy-2-thio-pseudouridine. In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2- thio-zebularine, 2-thio-zebularine, 2-methoxy-cytidine, 2-methoxy-5-methyl-cytidine, 4-methoxy-pseudoisocytidine, and 4-methoxy-I-methyl-pseudoisocytidine.

In other embodiments, modified nucleosides include 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl- guanosine, 7-methylinosine, 6-methoxy-guanosine, 1-methylguanosine, N2- methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio- guanosine.

In some embodiments, the nucleotide can be modified on the major groove face and can include replacing hydrogen on C-5 of uracil with a methyl group or a halo group.

In specific embodiments, a modified nucleoside is 5'-O-(1-Thiophosphate)-Adenosine, 5'-O-(1-Thiophosphate)-Cytidine, 5'-O-(1-Thiophosphate)-Guanosine, 5'-O-(1-Thiophosphate)-Uridine or 5'-O-(1-Thiophosphate)-Pseudouridine.

In further specific embodiments the modified RNA may comprise nucleoside modifications selected from 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha-thio-uridine, 4-thio-uridine, 6-aza-uridine, 5-hydroxy-uridine, deoxy-thymidine, 5-methyl-uridine, Pyrrolo-cytidine, inosine, alpha-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2 -amino-purine, Pseudo-iso-cytidine, 6-Chloro-purine, N6-methyl-adenosine, cc-thio-adenosine, 8-azido-adenosine, 7-deaza-adenosine.

The target RNA to be produced in the process according to the present invention is broadly defined as follows and can include the following types of RNA.

Preferred target RNAs are defined below and include mRNA, viral RNA, retroviral RNA and replicon RNA, bicistronic or multicistronic RNA, small interfering RNA (siRNA), antisense RNA, CRISPR RNA, ribozymes, aptamers, riboswitches, immunostimulating RNA, transfer RNA (tRNA), ribosomal RNA (rRNA), small nuclear RNA (snRNA), small nucleolar RNA (snoRNA), microRNA (miRNA), and Piwi-interacting RNA (piRNA). Very preferred target RNA is immunostimulating RNA and/or mRNA. Most preferred target RNA is mRNA.

*In vivo,* transcription of DNA usually results in the so-called premature RNA, which has to be processed into so-called messenger RNA, usually abbreviated as mRNA. Processing of the premature RNA, e.g. in eukaryotic organisms, comprises a variety of different post-transcriptional modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, an mRNA found in eukaryotic cells comprises a 5'-cap, optionally a 5'UTR, an open reading frame, optionally a 3'UTR and a poly(A) sequence. If the RNA is obtained from bacterial cells, the mRNA has to be enzymatically capped by a capping enzyme. Suitable capping enzymes are known in the art. Aside from messenger RNA, several non-coding types of RNA exist which may be involved in regulation of transcription and/or translation.

A "5 '-cap" is an entity, typically a modified nucleotide entity, which generally "caps" the 5'-end of a mature mRNA.. A 5'-cap may typically be formed by a modified nucleotide (cap analog), particularly by a derivative of a guanine nucleotide. Preferably, the 5'-cap is linked to the 5'-terminus of a nucleic acid molecule, preferably an RNA, via a 5'-5'-triphosphate linkage. A 5'-cap may be methylated, e.g. m7GpppN (e.g. m7G(5')ppp(5')G (m7G)), wherein N is the terminal 5' nucleotide of the nucleic acid carrying the 5'-cap, typically the 5'-end of an RNA. Such a 5'-cap structure is called cap0. *In vivo,* capping reactions are catalyzed by capping enzymes. *In vitro,* a 5'-cap may be formed by a modified nucleotide, particularly by a derivative of a guanine nucleotide. Preferably, the 5'-cap is linked to the 5'-terminus via a 5'-5'-triphosphate linkage.

A 5'-cap may be methylated, e.g. m7GpppN, wherein N is the terminal 5' nucleotide of the nucleic acid carrying the 5'-cap, typically the 5'-end of an RNA. m7GpppN is the 5'-cap structure which naturally occurs in mRNA, typically referred to as capO structure.

Enzymes, such as cap-specific nucleoside 2'-O-methyltransferase enzyme create a canonical 5'-5'-triphosphate linkage between the 5'-terminal nucleotide of an mRNA and a guanine cap nucleotide wherein the cap guanine contains an N7 methylation and the 5'-terminal nucleotide of the mRNA contains a 2'-O-methyl. Such a structure is called the cap1 structure.

Further examples of 5'cap structures include glyceryl, inverted deoxy abasic residue (moiety), 4',5' methylene nucleotide, 1 -(beta-D- erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. Further modified 5'-CAP structures which may be used in the context of the present invention are CAP1 (methylation of the ribose of the adjacent nucleotide of m7GpppN), CAP2 (methylation of the ribose of the 2nd nucleotide downstream of the m7GpppN), CAP3 (methylation of the ribose of the 3rd nucleotide downstream of the m7GpppN), CAP4 (methylation of the ribose of the 4th nucleotide downstream of the m7GpppN), ARCA (anti-reverse CAP analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

A "5'-UTR" is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, which are also called regulatory elements. Such regulatory elements may be, for example, ribosomal binding sites. The 5'-UTR may be posttranscriptionally modified, for example by addition of a 5'-cap. In the context of the present invention, a 5'-UTR corresponds to the sequence of a mature mRNA, which is located between the 5'-cap and the start codon. Preferably, the 5'-UTR corresponds to the sequence, which extends from a nucleotide located 3' to the 5'-cap, preferably from the nucleotide located immediately 3' to the 5'-cap, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence, which corresponds to such RNA sequence. In the context of the present invention, the term "a 5'-UTR of a gene" is the sequence, which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence of the 5'-UTR.

Generally, the term "3'-UTR" refers to a part of the nucleic acid molecule, which is located 3' (i.e. "downstream") of an open reading frame and which is not translated into protein. Typically, a 3'-UTR is the part of an mRNA, which is located between the protein coding region (open reading frame (ORF) or coding sequence (CDS)) and the poly(N/A) sequence of the (m)RNA. In the context of the invention, a 3'-UTR of the nucleic acid molecule may comprise more than one 3'-UTR elements, which may be of different origin, such as sequence elements derived from the 3'-UTR of several (unrelated) naturally occurring genes. Accordingly, the term 3'-UTR may also comprise elements, which are not encoded in the template, from which an RNA is transcribed, but which are added after transcription during maturation, e.g. a poly(A) sequence. A 3'-UTR of the mRNA is not translated into an amino acid sequence. The 3'-UTR sequence is generally encoded by the gene, which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excize optional introns and modifications of the 3'-end, such as polynucleotidylation/polyadenylation of the 3'-end of the pre-mature mRNA and optional endo-/ or exonuclease cleavages etc. In the context of the present invention, a 3'-UTR corresponds to the sequence of a mature mRNA which is located between the stop codon of the protein coding region, preferably immediately 3' to the stop codon of the protein coding region, and the poly(A) sequence of the mRNA. The term "corresponds to" means that the 3'-UTR sequence may be an RNA sequence, such as in the mRNA sequence used for defining the 3'-UTR sequence, or a DNA sequence, which corresponds to such RNA sequence. In the context of the present invention, the term "a 3'-UTR of a gene" is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR. As used herein, the term "3'-UTR element" typically refers to a fragment of a 3'-UTR as defined herein. In particular, the term comprises any nucleic acid sequence element, which is located 3' to the ORF in the artificial nucleic acid molecule, preferably the mRNA, according to the invention. Accordingly, the term covers, for example, sequence elements derived from the 3'-UTR of a heterologous gene as well as elements such as a poly(C) sequence or a histone stem-loop.

Polyadenylation is typically understood to be the addition of a poly(A) sequence, i.e. a sequence of adenosine monophosphate, to a nucleic acid molecule, such as an RNA molecule. As used in the context of the present invention, the term may relate to polyadenylation of RNA as a cellular process as well as to polyadenylation carried out by enzymatic reaction *in vitro* or by chemical synthesis.

A poly(A) sequence, also called poly(A) tail or 3'-poly(A) tail, is usually understood to be a sequence of adenine nucleotides, e.g., of up to about 400 adenosine nucleotides, e.g. from about 20 to about 400, preferably from about 50 to about 400, more preferably from about 50 to about 300, even more preferably from about 50 to about 250, most preferably from about 60 to about 250 adenosine nucleotides, which is preferably added to the 3'-terminus of an mRNA. A poly(A) sequence is typically located at the 3'-end of an RNA, in particular mRNA. In the context of the present invention, a poly(A) sequence may be located within an (m)RNA or any other nucleic acid molecule, such as, e.g., in a vector, for example, in a vector serving as template for the generation of an RNA, preferably an mRNA, e.g., by transcription of the vector. In the context of the present invention, the term 'poly(A) sequence' further comprises also sequence elements, preferably artificial sequence elements, that are part of the 3'-UTR or located at the 3'-terminus of the artificial nucleic acid molecule, and which preferably comprise up to 1100 adenine nucleotides, more preferably at least 60, 70, 80, 90, 100, 110, 120, 130, 140, 150, 160, 170, 180, 190, 200, 210, 220, 230, 240, 250, 300, 350, 400, 500, 600, 700, 800, 900, or at least 1000 adenine nucleotides. In general, the poly(A) sequence consists of adenosine monophosphates.

The term "RNA" as used herein also encompasses other RNA molecules, such as viral RNA, i.e. RNA derived from a virus, retroviral RNA, i.e. RNA derived from a retrovirus, and replicon RNA.

The term "bicistronic" or "multicistronic" RNA as used herein is typically RNA, preferably an mRNA, that typically has two (bicistronic) or more (multicistronic) open reading frames (ORF). An open reading frame in this context is a sequence of codons that is translatable into a peptide or protein.

The term "small interfering RNA" (siRNA) as used herein refers to double-stranded RNA molecules which are from about 10 to about 30 nucleotides long and which are named for their ability to specifically interfere with protein expression. Preferably, siRNA molecules are 12-28 nucleotides long, more preferably 15-25 nucleotides long, still more preferably 19-23 nucleotides long and most preferably 21-23 nucleotides long. Therefore, preferred siRNA molecules are 12, 13, 14, 15, 16, 17, 18, 19, 20, 21, 22, 23, 24, 25, 26, 27 28 or 29 nucleotides in length.

The term "antisense RNA" includes any RNA molecule which is suitable as an antisense RNA to regions of RNA, preferably mRNA, and most preferably to regulatory elements thereof, and which is capable of causing inhibition of gene expression by complementary binding to these regions. The antisense RNA may also comprise DNA sequences.

The term "CRISPR RNA" is derived from CRISPRs (Clustered Regularly Interspaced Short Palindromic Repeats) evolved in bacteria as an adaptive immune system to defend against viral attack. "Clustered Regularly Interspaced Short Palindromic Repeats" or "CRISPRs", as used herein refers to loci containing multiple short direct repeats that are found in the genomes of approximately 40% of sequenced bacteria and 90% of sequenced archaea. The CRISPR system is a microbial nuclease system involved in defense against invading phages and plasmids that provides a form of acquired immunity. Upon exposure to a virus, short segments of viral DNA are integrated into the CRISPR locus. "CRISPR RNA" is transcribed from a portion of the CRISPR locus that includes the viral sequence. That RNA, which contains sequence complementary to the viral genome, mediates targeting of a Cas9 protein to the sequence in the viral genome. The Cas9 protein cleaves and thereby silences the viral target. CRISPR clusters are transcribed and processed into mature CRISPR (Clustered Regularly Interspaced Short Palindromic Repeats) RNA (CRISPR RNA).

"Ribozymes" are typically RNA molecules having an enzymatic activity which is able to repeatedly cleave other separate RNA target molecules in a nucleotide base sequence specific manner, blocking their expression and affecting normal functions of other genes. Ribozymes can be targeted to virtually any RNA transcript, and achieve efficient cleavage in vitro.

The term "aptamers" is understood herein to refer to nucleic acid molecules having specific binding affinity to molecules through interactions other than classic Watson-Crick base pairing. The term "RNA aptamer" as used herein is an aptamer comprising ribonucleoside units. Aptamers, preferably RNA aptamers, are capable of specifically binding to selected targets and modulating the target's activity. Aptamers have a number of desirable characteristics for use as therapeutics and diagnostics including high specificity and affinity, biological efficacy, and excellent pharmacokinetic properties.

Riboswitches are RNA elements that reside in the 5' untranslated region (UTR) of genes and modulate their expression using either transcriptional or translational mechanisms (Roth and Breaker (2009) Annu Rev Biochem 78: 305-334).

The term "immunostimulating" RNA or "immunostimulatory" RNA (isRNA) as used herein may typically be an RNA that is able to induce an innate immune response. It usually does not have an open reading frame and thus does not provide a peptide-antigen or immunogen but elicits an immune response, e.g. by binding to a specific kind of Toll-like receptor (TLR) or other suitable receptors. However, of course also mRNAs having an open reading frame and coding for a peptide/protein may induce an innate immune response and, thus, may be immunostimulatory RNAs.

As used herein, the phrase "small nuclear RNA" (snRNA) refers to small RNA molecules which are synthesized and/or function in the nucleoplasm and/or the nucleolus of the cell.

The term "small nucleolar RNAs" (snoRNAs) are non-coding RNAs involved in RNA processing. There are two major subclasses of snoRNAs, termed box C/D and box H/ACA snoRNAs. These two classes contain guide sequences that are known to canonically pair with complementary regions on a target pre-rRNA,, forming a RNA duplex and facilitating the enzymatic activity of methylases and uridylases that site-specifically modify pre-rRNA bases by either 2'-0-methylation or pseudouridylation, respectively. These modifications of the rRNA are critical to ribosome assembly and viability.

"Micro RNAs" (miRNAs) play an important role in regulating gene activity. Micro-RNAs are single-stranded RNAs of typically 22-nucleotides that are processed from about 70 nucleotide hairpin RNA precursors by the RNase III nuclease. Similar to siRNAs, miRNAs can silence gene activity through destruction of homologous mRNA in plants or blocking its translation in plants and animals. These 20-22 nucleotide non-coding RNAs have the ability to hybridize via base-pairing with specific target mRNAs and downregulate the expression of these transcripts, by mediating either RNA cleavage or translational repression. Recent studies have indicated that miRNAs have important functions during development. Further, miRNAs are transcribed by RNA polymerase 11 as polyaclenylated and capped messages known as pri-miRNAs.

"Piwi-interacting RNA" (piRNA) is a class of small non-coding RNA molecules that is expressed in, or can be introduced into animal cells (see, e.g., Seto et al. (2007) Molecular Cell, 26(5): 603-609; Siomi et al. (2011) Nat. Rev. Mol. Cell. Biol., 12:246-258). piRNAs form RNA-protein complexes through interactions with piwi proteins. These piRNA complexes have been linked to both epigenetic and post-transcriptional gene silencing of retrotransposons and other genetic elements.

In a preferred embodiment of the present invention, the target RNA is mRNA and encodes a peptide or protein.

The target RNA to be produced by the *in vivo* transcription process according to the present invention can preferably encode a wide range of proteins or peptides.

In a preferred embodiment, the target RNA comprises at least one open reading frame, which encodes a therapeutic protein or peptide, or a fragment, variant or derivative thereof. In another embodiment, an antigen is encoded by the at least one open reading frame. Preferably, the antigen is a pathogenic antigen, a tumor antigen, an allergenic antigen or an autoimmune antigen. The administration of the target RNA encoding the antigen may be used in a genetic vaccination approach against a disease involving said antigen.

Fragments or parts of a protein in the context of the present invention are typically understood to be peptides corresponding to a continuous part of the amino acid sequence of a protein, preferably having a length of about 6 to about 20 or even more amino acids, e.g. parts as processed and presented by MHC class I molecules, preferably having a length of about 8 to about 10 amino acids, e.g. 8, 9, or 10, (or even 1 1 , or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. Fragments or parts of the proteins as defined herein may also comprise epitopes or functional sites of those proteins. Preferably, fragments or parts of a proteins in the context of the invention are antigens, particularly immunogens, e.g. antigen determinants (also called 'epitopes'), or do have antigenic characteristics, eliciting an adaptive immune response. Therefore, fragments of proteins or peptides may comprise at least one epitope of those proteins or peptides. Furthermore, also domains of a protein, like the extracellular domain, the intracellular domain or the transmembrane domain, and shortened or truncated versions of a protein may be understood to comprise a fragment of a protein.

In an alternative embodiment, an antibody is encoded by the at least one open reading frame of the target RNA according to the invention.

The term "open reading frame" (ORF) as used herein may typically be a sequence of several nucleotide triplets, which may be translated into a peptide or protein. An open reading frame preferably starts with a start codon, i.e. a combination of three subsequent nucleotides coding usually for the amino acid methionine (ATG or AUG), at its 5'-end and a subsequent region, which usually exhibits a length, which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG or AUG) and which preferably terminates with a stop codon (e.g., TAA, TGA, or TAG or UAA, UAG, UGA, respectively). The open reading frame may be isolated or it may be incorporated in a longer nucleic acid sequence, for example in a vector or an mRNA. An open reading frame may also be termed 'protein coding region'.

The protein that is encoded by the target RNA to be produced by the *in vivo* transcription process of the present invention is preferably an antigen. In the context of the present invention "antigen" refers typically to a substance, which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells.

More preferably, the target RNA according to the present invention may encode a protein or a peptide, which comprises a pathogenic antigen or a fragment, variant or derivative thereof.

Such pathogenic antigens are derived from pathogenic organisms, in particular bacterial, viral or protozoological (multicellular) pathogenic organisms, which evoke an immunological reaction in a subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Pathogenic antigens are peptide or protein antigens preferably derived from a pathogen associated with infectious disease, which are preferably selected from antigens derived from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli 01 57:H7, 01 1 1 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus (HBV), Hepatitis C Virus (HCV), Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV- 7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

The target RNA according to the present invention may encode a protein or a peptide, which relates to a tumor antigen.

In this further embodiment, the target RNA according to the present invention may encode a tumor antigen or a fragment, variant or derivative of said tumor antigen selected from the group consisting of 5T4, 707-AP, 9D7, AFP, AlbZIP HPG1, alpha-5- beta-1 -integrin, alpha-5-beta-6-integrin, alpha-actinin-4/m, alpha-methylacyl-coenzyme A racemase, ART-4, ARTC1 /m, B7H4, BAGE-1, BCL-2, bcr/abl, beta-catenin/m, BING-4, BRCAl/m, BRCA2/m, CA 1 5-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B'I, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT- V, gp100, GPC3, GPNMB/m, HAGE, HAST-2, hepsin, Her2/neu, HERV-K-MEL, HLA- A*0201 -R1 71, HLA-A1 1 /m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1 R, IL-13Ra2, IL-2R, IL-5, immature laminin receptor, kallikrein-2, kallikrein-4, KÏ67, IAA0205, KIAA0205/m, K -LC-1, K-Ras/m, LAGE-A1, LDLR-FUT, MAGE-A1, MAGE-A2, MAGE- A3, MAGE-A4, MAGE-A6, MAGE-A9, MAGE-A10, MAGE-A12, MAGE-B1, MAGE-B2, MAGE-B3, MAGE- B4, MAGE-B5, MAGE-B6, MAGE-B10, MAGE-B1 6, MAGE-B1 7, MAGE-C1, MAGE-C2, MAGE-C3, MAGE-D1, MAGE-D2, MAGE-D4, MAGE-E1, MAGE-E2, MAGE-F1, MAGE-HI, MAGEL2, mammaglobin A, MART-1/melan-A, MART-2, MART-2/m, matrix protein 22, MC1 R, M-CSF, MEl/m, mesothelin, MG50/PXDN, MMP1 1, MN/CA IX-antigen, MRP-3, MUC-1, MUC-2, MUM-l/m, MUM-2/m, MUM-3/m, myosin class l/m, NA88-A, N- acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p1 5, p1 90 minor bcr-abl, p53, p53/m, PAGE-4, PAI-1, PAI-2, PAP, PART-1, PATE, PDEF, Pim-1 - Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD1 68, RU1, RU2, S-1 00, SAGE, SART-1, SART-2, SA,RT-3, SCC, SIRT2/m, Sp1 7, SSX-1, SSX-2/HOM-MEL-40, SSX-4, STAMP-1, STEAP-1, survivin, survivin- 2B, SYT-SSX-1, SYT-SSX-2, TA-90, TAG-72, TARP, TEL-AML1, TGFbeta, TGFbetaRII, TGM- 4, TPI/m, TRAG-3, TRG, TRP-1, TRP-2/6b, TRP/INT2, TRP-p8, tyrosinase, UPA, VEGFR1, VEGFR-2/FLK-1, WT1 and a immunoglobulin idiotype of a lymphoid blood cell or a T cell receptor idiotype of a lymphoid blood cell.

In a preferred embodiment, the target RNA encodes a protein or a peptide, which comprises a therapeutic protein or a fragment, variant or derivative thereof.

Therapeutic proteins as defined herein are peptides or proteins, which are beneficial for the treatment of any inherited or acquired disease, or which improve the condition of an individual. Particularly, therapeutic proteins play a big role in the creation of therapeutic agents that could modify and repair genetic errors, destroy cancer cells or pathogen-infected cells, treat immune system disorders, treat metabolic or endocrine disorders, among other functions. For instance, the protein hormone erythropoietin (EPO) can be utilized in treating patients with erythrocyte deficiency, which is a common cause of kidney complications. Furthermore the terms adjuvant proteins and therapeutic antibodies are encompassed by therapeutic proteins as well as hormones used in replacement therapy, which is e.g. used in the therapy of women in menopause. Furthermore, therapeutic proteins may be used for other purposes, e.g. wound healing, tissue regeneration, angiogenesis, etc. Therefore therapeutic proteins can be used for various purposes including treatment of various diseases like e.g. infectious diseases, neoplasms (e.g. cancer or tumor diseases), diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, independently if they are inherited or acquired.

The present invention is also directed to a vector comprising a DNA sequence encoding the target RNA as defined above and a DNA sequence encoding an RNase inhibitor. The additional DNA sequence encoding an RNase inhibitor will allow producing recombinant RNase inhibitor in the microorganism or host cell producing the target RNA, thereby stabilizing the formed target RNA against degradation.

In a preferred embodiment of the present invention, the vector comprises a DNA sequence encoding an RNase inhibitor which is selected from *E.coli* RNase inhibitors RRaA and RraB and human RNase inhibitor RNH1. The *E.coli* RNase inhibitors RRaA and RraB are known to inhibit Ribonuclease E (RNase E) of *E.coli* (see Gao et al., (2006) Mol. Microbiol. 61(2): 394-406). Most preferred vectors comprise the DNA sequence encoding human RNase inhibitor RNH1.

The present invention is also directed to a microorganism comprising a vector as described above or a first vector comprising a DNA sequence encoding a target RNA and a second vector comprising a DNA sequence encoding an RNase inhibitor. The RNase inhibitor encoded by the vector of the host cell preferably is selected from *E.coli* RNase inhibitor RraA, *E.coli* RNase inhibitor RraB and human RNase inhibitor RNH1 and more preferably is human RNase inhibitor RNH1.

The present invention is also directed to the use of a microorganism comprising a vector comprising a DNA sequence encoding a target RNA for the *in vivo* transcription of said DNA sequence into said target RNA anda DNA sequence encoding an RNase inhibitor. In another preferred embodiment, the microorganism comprises a first vector comprising a DNA sequence encoding the target RNA and a second vector comprising a DNA sequence encoding an RNase inhibitor. In another preferred embodiment, the RNase inhibitor is selected from *E.coli* RNase inhibitor RraA and human RNase inhibitor RNH1.

The present invention is also directed to the use of an expression vector comprising a DNA sequence encoding an RNase inhibitor in a process for the *in vivo* transcription of a DNA sequence encoding a target RNA. In a preferred embodiment, the RNase inhibitor is selected from *E.coli* RNase inhibitor RraA and human RNase inhibitor RNH1.

### Examples

The following examples are merely illustrative and shall describe the present invention in a further way. These examples shall not be construed to limit the present invention thereto.

### Example 1: Cloning of vectors

The cloning of five different vector constructs IB-1, IB-3, IB-4, IB-5, IB-6 encoding the target RNA was carried out as follows:
For the target RNA to be transcribed *in vivo,* a non-coding DNA sequence of 500 base pairs (SEQ ID No. 1) corresponding to the target RNA was inserted into each vector construct. In vector constructs IB-5 and IB-6 the start codon was deleted and three additional stop codons were inserted to prevent any translation into recombinant protein.

The DNA sequence encoding the target RNA further included six restriction sites (3 on each end of the sequence (at the 5'-end: BGIII, Ndel, BamHI; at the 3'-end: XhoI, AvrII, BlpI).

In vector constructs IB-5 and IB-6, the DNA sequence encoding an RNase inhibitor was additionally inserted. Vector construct IB-5 comprised the DNA sequence of the RraA gene of *E.coli* strain K12 (Accession No POA8R0; UniProtKB; SEQ ID No. 3), vector construct IB-6 comprised the DNA sequence of the human RNase inhibitor gene RNH1 (Accession No P13489; UniProtKB; SEQ ID No. 4). Vector construct IB-1 (pCV19min-N500-T7-Term) contained a T7 promoter (SEQ ID No. 5), the DNA encoding the non-coding 500 bp target RNA sequence and a T7 terminator (SEQ ID No. 6).

Vector construct IB-3 (ptac-RNA500-rrnBT1T2) contained a tac promoter, the DNA sequence encoding the non-coding 500 bp target RNA sequence and a rrnBT1T2 terminator (SEQ ID No. 7).

Vector IB-4 (pET-21(+)-RNA500) contained a T7 promoter (SEQ ID No. 5), a lac operator (SEQ ID No. 8), the DNA sequence encoding; the non-coding 500 bp target RNA sequence and a T7 terminator (SEQ ID No. 6).

Vector IB-5 (pET-Duet-1-EccraA(Ec)-RNA500) contained a first T7 promoter (SEQ ID No. 5), a first lac operator (SEQ ID No. 8) and the DNA sequence encoding the non-coding, 500 bp target RNA sequence and a second T7 promote r(SEQ ID No. 5), a second lac operator (SEQ ID No. 8) and a DNA sequence encoding RNase inhibitor RraA of *E.coli* (SEQ ID No. 3)as well as a T7 terminator (SEQ ID No. 6).

Vector IB-6 (pET-Duet-1-HsRNH1(Ec)-RNA500) contained a first T7 promoter (SEQ ID No. 5), a first lac operator (SEQ ID No. 8) and the DNA sequence encoding the non-coding, 500 bp target RNA sequence and a second T7 promoter (SEQ ID No. 5), a second lac operator (SEQ ID No. 8) and a DNA sequence encoding the human RNase inhibitor HsRNH1 (SEQ ID No. 4) as well as a T7 terminator (SEQ ID No. 6).

The vector constructs IB1-, IB-3, IB-4, IB-5 and IB-6 were subsequently transformed into *E.coli* strains according to the following Table 1.

**Table 1: Transformation of E.coli strains wth vector constructs**

| Vector construct | Expression system | *E.coli* strain | RNase inhibitor | Transcript size |
|---|---|---|---|---|
| IB-1 | T7 | BL21(DE3) | - | 562 b |
| IB-3 | Tac | XL10-Gold | - | 466 b |
| IB-3 | Tac | SCS110 | - | 466 b |
| IB-4 | T71ac | BL21(DE3) | - | 517 b |
| IB-5 | T71ac | BL21(DE3) | EcRmA | 565 b |
| IB-6 | T71ac | BL21(DE3) | HsRNH1 | 565 b |

### Example 2: In vivo transcription of target RNA

*In vivo* transcription experiments were carried out with the transformed *E.coli* strains listed in Table 1 as follows.

The *E.coli cultures* in 20 ml LBₐₘₚ- medium were incubated overnight at 37°C and at 142 rpm. From these overnight cultures, 10 ml were used to prepare 50 ml production cultures by adding 40 ml fresh LBₐₘₚ medium and growing the cultures to OD₆₀₀ of 0.6. The cultures were induced by adding 0.5 mM and 1 mM IPTG, respectively, while the temperature was lowered to room temperature. Cultivation was continued for 2h, 4h, 5h, and 20h.

The RNA from the cultures was subsequently isolated using the RNeasy kit (Qiagen) according to the manufacturer's purification protocol.

Detection of the formed target RNA after *in vivo* transcription in *E.coli* was carried out by RT-PCR. The target RNA was converted in a first step into the complementary cDNA. In a second step the cDNA was further amplified.

The primers used in the RT-PCR and the subsequent PCR had the following sequences.
Reverse transcription:
   5'-GAT TGA CCG CGC CTC CTA TC-3' (SEQ ID No. 10)
PCR Fw:
   5'-AGC AAC AGC GTG CTA CAA GG-3' (SEQ ID No. 11)
PCR rv I:
   5'-TGC CCG CAC ATT GAG GAA AC-3' (SEQ ID No. 12)
PCR rv II:
   5'-GAG ATT GCC CCC GTG TAG-3' (SEQ ID No. 13)

The conditions for RT-PCR were as follows: 0.3 to 1 µg RNA; 1 µl primer (15 pmol), water ad 12 µl; 65°C, 5 min; Subsequently, the following reagents were added: 4 µl reaction buffer, 1µl of 200U/µl Revert Aid HMinus M-MulV-RT,1µl of Ribolock RNase inhibitor (20U/µl) and 2µl dNTP mix (10 mM); incubation time was 42°C at 1h followed by 70°C, 5 min to stop the reaction;

Conditions for the subsequent PCR were as follows: 2 µl product from reverse transcription, 25 µl PCR Master Mix (2x), 5 µl forward primer (5pmol), 5µl reverse primer (5pmol), 1.5 µl DMSO, 11.5 µl water;

The PCR scheme was as follows:

| Reaction step | Temperature (°C) | Time (min:sec) | Number of cycles |
|---|---|---|---|
| Initial Denaturation | 95 | 2:00 | 1 |
| Denaturation | 95 | 0:30 | 20 |
| Annealing | 65 | 0:30 | |
| Extension | 72 | 0:30 | |
| Completion | 72 | 10:00 | 1 |

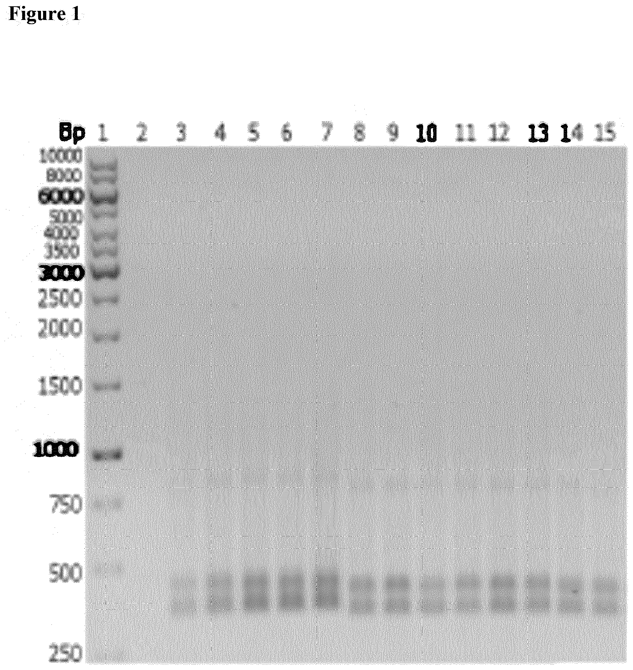

For each *in vivo* transcription experiment, the RT-PCR analysis was carried out twice. Analysis was carried out by agarose gel electrophoresis in order to identify the DNA product formed in the RT-PCR. As a positive control an *in vitro* transcribed product of IB-1 obtained according to standard protocols was used. As a negative control the endogenous total RNA of (non-transformed) *E.coli* was used. The test results are shown in Figure 1. The products analyzed in lanes 1 to 15 in Fig. 1 relate to the following test conditions:
1 - DNA size marker
2 - Negative control
3 - Positive control 12 ng
4 - Positive control 241 ng
5 - Positive control 603 ng
6 - Positive control 2410 ng
6 - RNA IB-5, 0.5 mM, 2h
7 - RNA IB-5, 1 mM, 2h
8 - RNA IB-5, 0.5 mM, 4h
9 - RNA IB-5, 1 mM, 4h
10 - RNA IB-5, 0.5 mM, 5h
11 - RNA IB-5, 1 mM, 5h
12 - RNA IB-5, 0.5 mM, 20h
12 - RNA IB-5, 1 mM, 20h

Similar RT-PCR results were obtained for the RNA analysis of the additional *E.coli* cultures with IB-1, IB-3, IB-4, and IB-6.

The band at about 355 bp relates to the target RNA to be expected from the *in vivo* transcribed construct IB-5.

For quantitative evaluation of the *in vivo* transcription tests the DNA bands in the agarose gel electrophoresis were further analyzed by the software Quantity One determining the intensity of the area of the band. In Figure 1 the corresponding two bands detected in most of the lanes in Figure 1 were considered as one common band. The additional software GraphPad Prism was used to generate a calibration function from the data set of the positive control. As calibration function a one and two phase association with y(0) = 0 was selected. The curve has a short linear increase followed by a plateau. The amounts of samples used for reverse transcription varied because the yield of total RNA obtained by the RNeasy kit also varied. Therefore, obtained amounts of target RNA product were determined relative to the total RNA amounts (ng target RNA / µg total RNA).

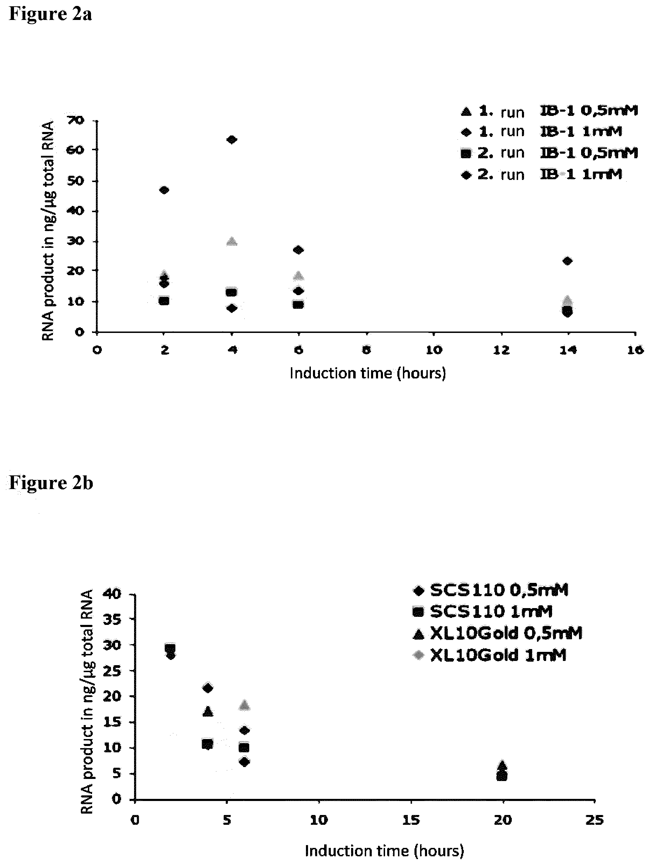

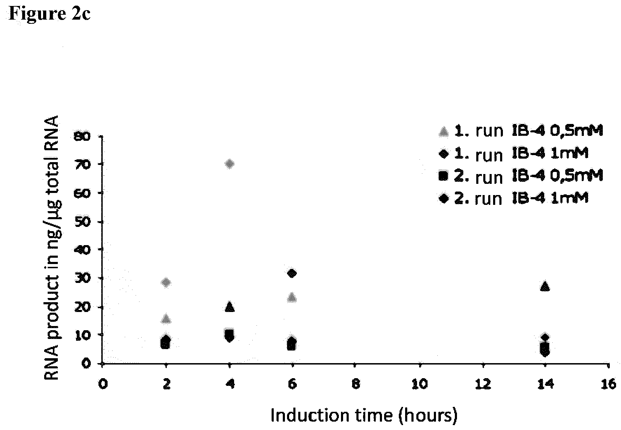

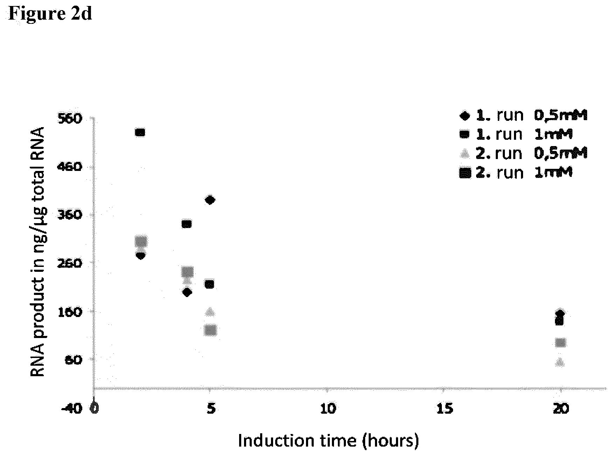

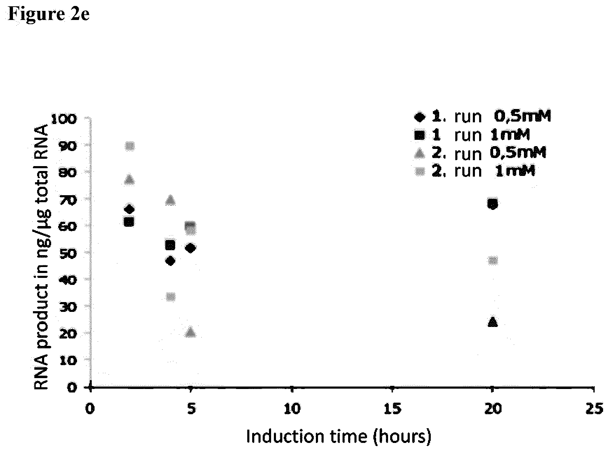

Based on the above quantification approach estimated amounts of target RNA were determined for the vector constructs IB-1 (see Figure 2a), IB-3 (see Figure 2b), IB-4 (see Figure 2c), IB-5 (see Figure 2d) and IB-6 (see Figure 2e) at the indicated concentrations of IPTG used for induction of *in vivo* transcription. For vector construct IB-3, two different *E.coli* strains SCS110 and XL10Gold were tested.

In summary, the following amounts of target RNA were calculated for the different vector constructs:

| | |
|---|---|
| IB-1, IB-3 and IB-4: | 5 to 50 ng/µg |
| IB-5: | 60 to 360 ng/µg |
| IB-6: | 20 to 90 ng/µg |

For IB-5, it was further calculated that the yield of 60 to 360 ng target RNA / µg total RNA corresponded to an overall yield of 1.5 mg target RNA per litre *E.coli* culture.

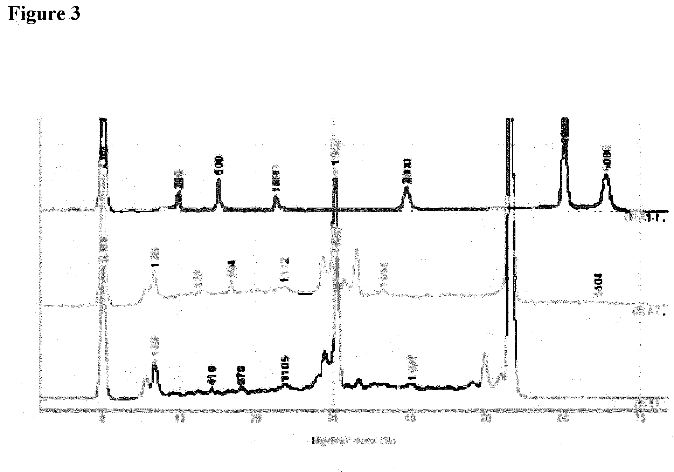

In order to support the above findings further analysis of the test samples obtained from *in vivo* transcription was carried out using chip electrophoresis. In Figure 3, electropherograms from samples derived from vector construct IB-5 were prepared using the MultiNA analyzer (Shimadzu). The top signal in Figure 3 refers to a size marker, the signal in the middle refers to the sample generated from IB-5, and the bottom signal refers to a negative control.

Figure 3 clearly shows the signal for the target RNA at 594 bp to be expected for vector construct IB-5 which is not found in the negative control thereby further confirming the formation of the target RNA transcript by the claimed *in vivo* transcription process.

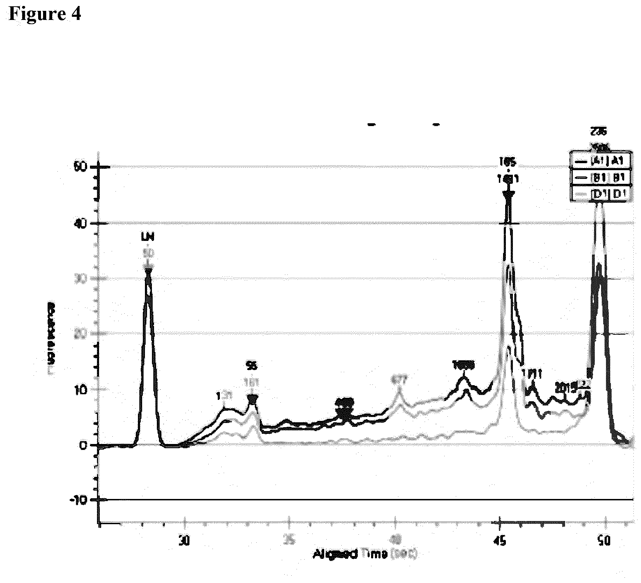

Alternative elctropherograms of the test samples obtained from *in vivo* transcription were prepared using a LabChip GX (Caliper Lifescience). The top signal in Figure 4 refers to a sample obtained by *in vivo* transcription of vector construct IB-5 at 0.5 mM IPTG, 5h. The signal in the middle refers to a sample obtained by *in vivo* transcription of vector construct IB-5 at 1 mM IPTG, 5h, and the bottom signal refers to a negative control obtained from total RNA of non-transformed *E.coli.* The samples generated from IB-5 show a signal for 677 bp while this signal is missing in the negative control.

In conclusion, electropherograms generated using two different types of instruments (MultiNA and LabChip GX) allowed the detection and identification of the expected target RNA transcript generated by the *in vivo* transcription process of the present invention.

Further, a similar analysis using chip electrophoresis (LabChip GX, Caliper) was carried out after additional depletion of the ribosomal RNA.

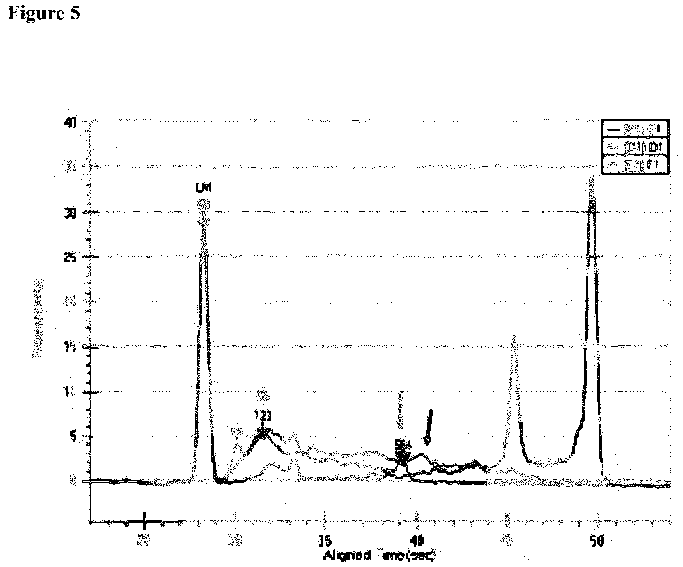

In Figure 5, a positive control (E1) containing *in vitro* transcribed vector construct IB-1 and total RNA of non-transformed *E.coli* is shown together with RNA isolated after *in vivo* transcription of vector construct IB-5 (F1). Both samples were additionally depleted of ribosomal RNA using the Microbe Express kit (Ambion) according to the manufacturer's protocol. Such depletion of ribosomal RNA was not done in the negative control (total RNA of non-transformed *E.coli*; D1) in which 16S and 23S RNA consequently still represent the main fractions.

### Example 3: Detection of the target RNA by Northern Blot and Dot Blot

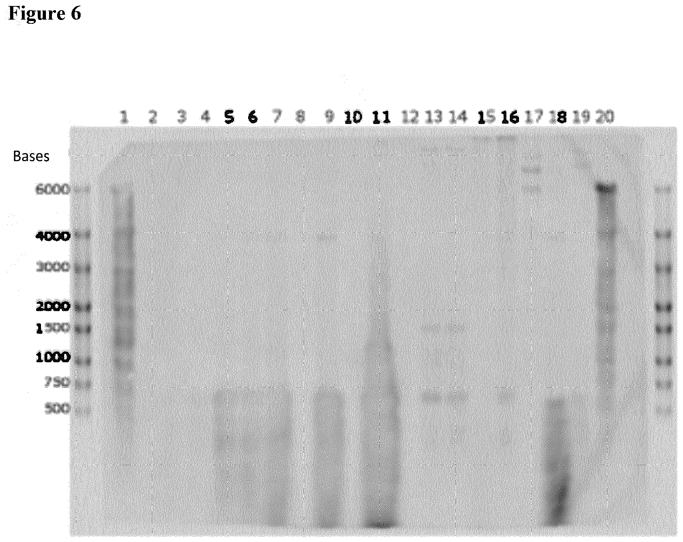

For a sequence-specific detection of the *in vivo* transcribed target RNA, northern blotting was carried out using a DNA probe of vector construct IB-1. The probe was labeled with dioxigenin using the PCR DIG Probe Synthesis Kit (Roche). As a positive control, *in vitro* transcribed vector construct IB-1 was used. As negative control total RNA of *E.coli* was used. Figure 6 shows the result of the Northern Blot with the following lanes.
1 - size marker
2 - positive control 0.1 ng
3 - positive control 1 ng
4 - positive control 5 ng
5 - positive control 50 ng
6 - positive control 100 ng
7 - positive control 250 ng
8 - no sample
9 - positive control 500 ng
10 - no sample
11 - positive control 1000 ng
12 - no sample
13 - RNA sample generated from vector construct IB-5 (0.5 mM IPTG, 5h)
14 - RNA sample generated from vector construct IB-5 (1 mM IPTG, 5h)
15 - RNA sample generated from vector construct IB-6 (0.5 mM IPTG, 4h)
16 - RNA sample generated from vector construct IB-6 (1 mM IPTG, 4h)
17 - negative control
18 - positive control with total RNA of *E.coli* after depletion of rRNA
19 - RNA sample generated from vector construct IB-5 after depletion of rRNA
20 - size marker

The expected band (547 bp) for the *in vitro* generated RNA transcript is present in the samples of the positive control (see lanes 2 to 7, 9 and 11). Lanes 13 and 14 reveal a band for the *in vivo* generated target RNA of vector construct IB-5 having a length of 565 bp. At least lane 16 further reveals the *in vivo* generated target RNA of vector construct IB-5 having a length of 565 bp. In lane 19 the same band is present after depletion of rRNA.

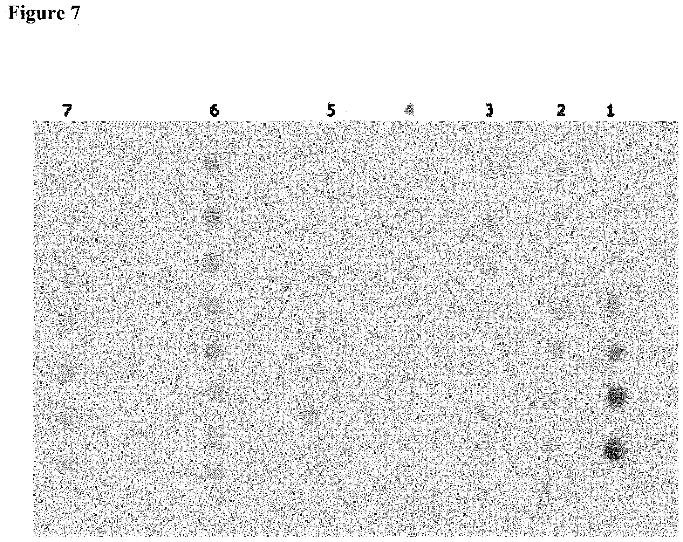

Additionally, a dot blot was carried out using an amount of RNA of 3 ng for each sample. As positive control, the *in vitro* transcribed RNA of vector construct IB-1 was used. Figure 7 shows the result of the dot blot.
Lane 1 (from top to bottom): positive control; 0.01 ng, 0.05 ng, 0.1 ng, 0.5 ng, 1 ng, 5 ng, 10 ng of IPTG.
Lane 2 (from top to bottom): vector construct IB-1; 0.5mM, 2h; 1mM, 2h; 0.5 mM, 4h; 1 mM, 4h; 0.5 mM, 6h; 1 mM, 6h; 0.5 mM, 20h; 1 mM, 20h;
Lane 3 (from top to bottom): vector construct IB-4; 0.5mM, 2h; 1mM, 2h; 0.5 mM, 4h; 1 mM, 4h; 0.5 mM, 6h; 1 mM, 6h; 0.5 mM, 20h; 1 mM, 20h;
Lane 4 (from top to bottom): vector construct IB-3, SCS110 cells; 0.5 mM, 2h; 1 mM, 2h; 0.5 mM, 4h; 1 mM, 4h; 1 mM, 6h; 0.5 mM, 20h; 1 mM, 20h;
Lane 5 (from top to bottom): vector construct IB-3, XL10 Gold cells; 0.5mM, 2h; 1mM, 2h; 0.5 mM, 4h; 1 mM, 4h; 0.5 mM, 6h; 1 mM, 6h; 0.5 mM, 20h; 1 mM, 20h;
Lane 6 (from top to bottom): vector construct IB-5; 0.5mM, 2h; 1mM, 2h; 0.5 mM, 4h; 1 mM, 4h; 0.5 mM, 5h; 1 mM, 5h; 0.5 mM, 20h; 1 mM, 20h;
Lane 7 (from top to bottom): vector construct IB-6; 0.5mM, 2h; 1mM, 2h; 0.5 mM, 4h; 0.5 mM, 5h; 1 mM, 5h; 0.5 mM, 20h; 1 mM, 20h;

The most significant dots are seen for the samples derived from vector constructs IB-5 and IB-6, with the samples generated from vector construct IB-5 showing the highest intensity in the dot blot.

For quantitative evaluation of the dots in Figure 7, the additional software GraphPad Prism was used as described above, allowing the calculation of the following amounts of target RNA generated by *in vivo* transcription from the corresponding vector constructs:

| Vector construct (cells) | Average yield of target RNA (µg/L) | Relative amount (%) (relative to IB-5) |
|---|---|---|
| IB-1 | 1345 | 24.6 |
| IB-3 (SCSI 10 cells) | 836 | 15.3 |
| IB-3 (XL10 Gold cells) | 277 | 5.1 |
| IB-4 | 248 | 17.3 |
| IB-5 | 5474 | 100 |
| IB-6 | 1331 | 24.3 |

It was further calculated for IB-5 that the yield of 60 to 360 ng target RNA / µg total RNA corresponded to a yield of 1.5 mg target RNA per litre *E.coli* culture.

Preferred embodiments of the present invention relate to:
1. Process for producing a target RNA in a host cell comprising the steps of:
   a) providing (i) a host cell comprising a vector comprising a DNA sequence encoding the target RNA and a DNA sequence encoding an RNase inhibitor or (ii) a host cell comprising a first vector comprising a DNA sequence encoding the target RNA and a second vector comprising a DNA sequence encoding an RNase inhibitor,
   b) fermenting the host cell and allowing the DNA sequence to be transcribed into the target RNA,
   c) obtaining the target RNA from the host cell.
2. Process according to embodiment 1, wherein the host cell is a bacterial cell.
3. Process according to embodiment 1 or 2, wherein the host cell is *E.coli.*
4. Process according to any one of embodiments 1 to 3, wherein the target RNA is selected from non-modified RNA and modified RNA, wherein the modified RNA comprises at least one modified nucleotide.
5. Process according to any one of embodiments 1 to 4, wherein the target RNA is selected from the group consisting of mRNA, viral RNA, retroviral RNA and replicon RNA, bicistronic or multicistronic RNA, small interfering RNA (siRNA), antisense RNA, CRISPR RNA, ribozymes, aptamers, riboswitches, immunostimulating RNA, ribosomal RNA (rRNA), small nuclear RNA (snRNA), small nucleolar RNA (snoRNA), microRNA (miRNA), and Piwi-interacting RNA (piRNA).
6. Process according to any one of embodiments 1 to 5, wherein the target RNA has a length of at least 100 nucleotides.
7. Process according to any one of embodiments 1 to 5, wherein the target RNA is an mRNA and codes for at least one antigen, a therapeutic protein, an antibody, a B cell receptor, a T cell receptor or a fragment, variant or derivative thereof.
8. Process according to embodiment 6, wherein the antigen is a tumor antigen, a pathogenic antigen, an allergenic antigen or an autoimmune antigen.
9. Process according to any one of embodiments 1 to 8, wherein the RNase inhibitor is selected from *E.coli* RNase inhibitor RraA and human RNase inhibitor RNH1.
10. Process according to any one of embodiments 1 to 9, wherein in step b) a compound capable of inhibiting peptide and/or protein synthesis is added.
11. Process according to embodiment 10, wherein the compound is chloramphenicol.
12. Process according to any one of embodiments 1 to 11, wherein in step b) a substance capable of inhibiting endogenous RNA polymerase is added.
13. Process according to embodiment 12, wherein the substance is a rifamycin.
14. Process according to embodiment 13, wherein the rifamycin is rifampicin.
15. Process according to any one of embodiments 1 to 14, wherein the target RNA is obtained from the host cell by separating the target RNA from the endogenous RNA of the host cell.
16. Process according to any one of embodiments 1 to 15, wherein obtaining the target RNA comprises a step of depleting the ribosomal RNA of the host cell.
17. Process according to embodiment 16, wherein the ribosomal RNA of the host cell is depleted by capture hybridization of the ribosomal RNA with complementary oligonucleotides immobilized on a solid phase.
18. Process according to any one of embodiments 1 to 17, wherein the target RNA is obtained by hybridization with a complementary nucleic acid sequence.
19. Process according to embodiment 18, wherein the complementary nucleic acid sequence is immobilized on a solid matrix.
20. Process according to any one of embodiments 1 to 19, wherein the target RNA comprises an affinity tag capable of binding to an affinity matrix.
21. Process according to embodiment 20, wherein the affinity tag comprises an aptamer.
22. Process according to embodiment 21, wherein the aptamer is capable of binding to Sephadex.
23. Process according to embodiment 20, wherein the affinity tag comprises an RNA sequence capable of binding to a protein and/or peptide.
24. Process according to embodiment 23, wherein the protein and/or peptide is a boxB RNA binding peptide.
25. Process according to any one of embodiments 20 to 24, wherein the target RNA further comprises a ribozyme sequence.
26. Process according to any one of embodiments 20 to 25, wherein the target RNA is obtained by binding of the affinity tag to an affinity matrix.
27. Process according to any one of embodiments 25 or 26, wherein the affinity tag is cleaved from the target RNA by activating the ribozyme sequence.
28. Vector comprising a DNA sequence encoding a target RNA and a DNA sequence encoding an RNase inhibitor.
29. Vector according to embodiment 28, wherein the RNase inhibitor is selected from *E.coli* RNase inhibitor RraA and human RNase inhibitor RNH1.
30. Vector according to embodiment 28 or 29, wherein the target RNA comprises an affinity tag.
31. Vector according to embodiment 30, wherein the affinity tag is an aptamer.
32. Vector according to embodiment 31, wherein the aptamer is capable of binding to Sephadex.
33. Vector according to any one of embodiments 30 to 32, wherein the target RNA further comprises a ribozyme sequence.
34. Vector according to any one of embodiments 28 to 33, wherein the DNA sequence encoding the target RNA is under the control of a promoter selected from the group consisting of T3 promoter, T7 promoter, sp6 promoter and tac promoter.
35. Vector according to any one of embodiments 28 to 34, further comprising a terminator.
36. Vector according to embodiment 35, wherein the terminator is selected from the T7 terminator, the VSV terminator, the PTH terminator, the *rrnB* T1 downstream terminator, the *rrnC* terminator, the concatemer junction sequence of the replicating T7 DNA,the rmBT1T2 terminator and a variant of any of the foregoing.
37. Host cell comprising the vector according to any one of embodiments 28 to 36.
38. Host cell comprising a first vector comprising a DNA sequence encoding a target RNA and a second vector comprising a DNA sequence encoding an RNase inhibitor.
39. Host cell according to embodiment 38, wherein the RNase inhibitor is selected from *E.coli* RNase inhibitor RraA and human RNase inhibitor RNH1.
40. Host cell according to any one of embodiments 37 to 39, wherein the host cell is a bacterium.
41. Host cell according to embodiment 40, wherein the bacterium is *E.coli.*
42. Host cell according to embodiment 41, wherein *E.coli* is capable of expressing a recombinant polymerase.
43. Host cell according to embodiment 42, wherein the recombinant polymerase is selected from T3 polymerase, T7 polymerase and sp6 polymerase.
44. Host cell according to any one of embodiments 41 to 43, wherein the *E.coli* is selected from the group consisting of DH5α, BL21, JM109, HMS174, B834, SCS 110, XL1 Blue and XL10 Gold.
45. Host cell according to any one of embodiments 41 to 43, wherein the *E.coli* is selected from the group consisting of BL21(DE3), JM109(DE3), HMS174(DE3) and B834(DE3).
46. Host cell according to any one of embodiments 41 to 45, wherein expression of one or more proteins is under the control of an operator.
47. Host cell according to embodiment 46, wherein the operator is the lac operator.
48. Use of a host cell according to any one of embodiments 37 to 47 for the *in vivo* transcription of said DNA sequence into said target RNA.
49. Use of an expression vector comprising a DNA sequence encoding an RNase inhibitor in a process for the *in vivo* transcription of a DNA sequence encoding a target RNA.
50. Use according to embodiment 49, wherein the RNase inhibitor is selected from *E.coli* RNase inhibitor RraA and human RNase inhibitor RNH1.

## Claims

1. A process for producing a target RNA in a host cell comprising the steps of:
a) providing a host cell comprising a vector comprising a DNA sequence encoding the target RNA,
b) fermenting the host cell and allowing the DNA sequence to be transcribed into the target RNA,
c) obtaining the target RNA from the host cell, wherein the target RNA is obtained from the host cell by separating the target RNA from the endogenous RNA of the host cell,
wherein a DNA sequence encoding an RNase inhibitor is integrated into the genome of the host cell.

2. The process according to claim 1, wherein the DNA sequence encoding the RNase inhibitor is recombinantly integrated into the genome of the host cell.

3. The process according to claim 1 or claim 2, wherein the host cell is a bacterial cell, and/or the RNase inhibitor is a human RNase inhibitor, preferably wherein the RNase inhibitor is human RNase inhibitor RNH1.

4. The process according to claim 1 or claim 2, wherein the host cell is selected from a yeast cell, a fungal cell, a plant cell or a eukaryotic cell; and/or the RNase inhibitor is a bacterial RNase inhibitor, preferably wherein the RNase inhibitor is E. *coli* RNase inhibitor RraA.

5. The process according to any one of claims 1 to 3, wherein the host cell is a bacterial cell selected from Escherichia coli (*E.coli*), Corynebacterium, Lactococcus lactis, Streptomyces, Bacillus subtilis and Pseudomonas fluorescens, preferably wherein the host cell is an *E.coli* cell.

6. The process according to any one of claims 1 to 5, wherein
(i) the target RNA is selected from non-modified RNA and modified RNA, wherein the modified RNA comprises at least one modified nucleotide;
(ii) the target RNA is selected from the group consisting of mRNA, viral RNA, retroviral RNA and replicon RNA, bicistronic or multicistronic RNA, small interfering RNA (siRNA), antisense RNA, CRISPR RNA, ribozymes, aptamers, riboswitches, immunostimulating RNA, ribosomal RNA (rRNA), small nuclear RNA (snRNA), small nucleolar RNA (snoRNA), microRNA (miRNA), and Piwi-interacting RNA (piRNA);
(iii) the target RNA has a length of at least 100 nucleotides; and/or
(iv) the target RNA is an mRNA and codes for at least one antigen, a therapeutic protein, an antibody, a B cell receptor, a T cell receptor or a fragment, variant or derivative thereof, optionally wherein the antigen is a tumor antigen, a pathogenic antigen, an allergenic antigen or an autoimmune antigen.

7. The process according to any one of claims 1 to 6, wherein
(i) in step b) a compound capable of inhibiting peptide and/or protein synthesis is added, optionally wherein the compound is chloramphenicol; and/or
(ii) in step b) a substance capable of inhibiting endogenous RNA polymerase is added, optionally wherein the substance is a rifamycin, optionally wherein the rifamycin is rifampicin.

8. The process according to any one of claims 1 to 7, wherein
(i) obtaining the target RNA comprises a step of depleting the ribosomal RNA of the host cell, optionally wherein the ribosomal RNA of the host cell is depleted by capture hybridization of the ribosomal RNA with complementary oligonucleotides immobilized on a solid phase; and/or
(ii) the target RNA is obtained by hybridization with a complementary nucleic acid sequence, optionally wherein the complementary nucleic acid sequence is immobilized on a solid matrix.

9. The process according to any one of claims 1 to 8, wherein the target RNA comprises an affinity tag capable of binding to an affinity matrix, optionally wherein the affinity tag comprises an aptamer, optionally wherein the aptamer is capable of binding to Sephadex.

10. The process according to claim 9, wherein the affinity tag comprises an RNA sequence capable of binding to a protein and/or peptide, optionally wherein the protein and/or peptide is a boxB RNA binding peptide.

11. The process according to claim 9 or claim 10, wherein
(i) the target RNA further comprises a ribozyme sequence; and/or
(ii) the target RNA is obtained by binding of the affinity tag to an affinity matrix;
optionally wherein the affinity tag is cleaved from the target RNA by activating the ribozyme sequence.

12. A host cell comprising a DNA sequence encoding an RNase inhibitor, wherein said DNA sequence is integrated into the genome, optionally wherein the RNase inhibitor is selected from *E.coli* RNase inhibitor RraA and human RNase inhibitor RNH1.

13. The host cell according to claim 12, comprising a vector comprising a DNA sequence encoding a target RNA, wherein the target RNA comprises an affinity tag, wherein the affinity tag is an aptamer, optionally wherein the aptamer is capable of binding to Sephadex and optionally wherein the target RNA further comprises a ribozyme sequence.

14. The host cell according to claim 13,
(i) wherein the DNA sequence encoding the target RNA is under the control of a promoter selected from the group consisting of T3 promoter, T7 promoter, sp6 promoter and tac promoter; and/or
(ii) wherein the vector further comprises a terminator, optionally wherein the terminator is selected from the T7 terminator, the VSV terminator, the PTH terminator, the rrnB T1 downstream terminator, the rrnC terminator, the concatemer junction sequence of the replicating T7 DNA, the rrnBT1T2 terminator and a variant of any of the foregoing.

15. Use of the host cell according to claim 13 or 14 for the *in vivo* transcription of said vector comprising a DNA sequence encoding a target RNA into said target RNA.


SEQUENCE LISTING

<110>  CureVac AG

<120>  Process for the in vivo production of RNA in a host cell

<130>  C09827WOEPD1

<160>  13

<170>  PatentIn version 3.5

<210>  1
<211>  500
<212>  DNA
<213>  artificial

<220>
<223>  insert for RNA production

<400>  1
agatctcata tgtgataata aggatccgga gcattgcccg cacattgagg aaaccgagtt      60

gcatatctca gagtattggc ccccgtgtag gttattcttg acagacagtg gagcttattc     120

actcccagag tcgcatacta cggtactggt gacagaccta cgtcgtcagt tgaccagtcc     180

gccactagac gtgagtccgt caaagcagtt agaagttaca ctctattcgg attacagctc     240

gaaggagcag gagtagtgtt cttgctctaa gtaccgagtg tgcccaatac ccgatcagct     300

tattaacgaa cggctcctcc tcttagacgt aagtgcggaa tctggggatc aaattactga     360

ctgcctggat taccctcgga catataacct tgtagcacgc tgttgctgta taggtgacca     420

acgcccatag accagctctc ttagtccgga caacgatagg aggcgcggtc aatctacttc     480

tctcgagcct agggctgagc                                                 500


<210>  2
<211>  547
<212>  DNA
<213>  artificial

<220>
<223>  insert for RNA production with stop codon

<400>  2
gggagaaagc tcaagcttgg agcaatgccc gcacattgag gaaaccgagt tgcatatctc      60

agagtattgg cccccgtgta ggttattctt gacagacagt ggagcttatt cactcccagg     120

atccgagtcg catactacgg tactggtgac agacctaggt cgtcagttga ccagtccgcc     180

actagacgtg agtccgtcaa agcagttaga tgttacactc tattagatct cggattacag     240

ctggaaggag caggagtagt gttcttgctc taagtaccga gtgtgcccaa tacccgatca     300

gcttattaac gaacggctcc tcctcttaga ctgcagcgta agtgcggaat ctggggatca     360

aattactgac tgcctggatt accctcggac atataacctt gtagcacgct gttgctgtat     420

aggtgaccaa cgcccactcg agtagaccag ctctcttagt ccggacaatg ataggaggcg 480

cggtcaatct acttctggct agttaagaat aggctgcacc gacctctata agtagcgtgt 540

cctctag 547

<210> 3
<211> 486
<212> DNA
<213> Escherichia coli

<400> 3
atgaaatacg acacctctga attatgtgac atttaccagg aagatgttaa tgttgttgaa 60

cctctttttt ctaactttgg gggacgcgct agctttggtg gtcaaattat tactgttaaa 120

tgttttgaag ataatggact tttatatgac ttacttgaac aaaatggacg tggacgggtg 180

cttgtggtag atgggggggg gagtgtgcgt cgtgctttgg tggatgcaga attagcacgt 240

ttagctgtac aaaatgaatg ggaagggtta gtaatttatg gagcggtacg tcaagtggac 300

gatttggaag aattagatat tggtattcaa gcaatggcgg ctatcccagt aggagcggcg 360

ggagaaggaa ttggagaatc ggatgtacgg gttaactttg gcggtgtaac ttttttttca 420

ggcgatcatc tgtatgctga caacacaggt attattttaa gtgaagatcc tttggacatt 480

gaataa 486

<210> 4
<211> 1410
<212> DNA
<213> Homo sapiens

<400> 4
atgtctcttg atattcaatc cctggatatt cagtgtgaag aactttctga tgctcgttgg 60

gcagaactcc tccctttact ccagcaatgc caagttgtcc gtcttgatga ttgtggttta 120

accgaagccc gctgcaaaga tatctcatcc gcgttacggg tcaatccagc gctggccgaa 180

ttgaatctgc gcagtaatga actgggcgat gtaggtgttc actgtgtttt acaaggctta 240

cagaccccat cgtgcaaaat tcaaaaactc tcccttcaaa actgttgcct cacaggagcc 300

ggctgtggcg tcttgtcatc aactttacgt accctcccta ccttacaaga attacatctg 360

tcagacaact tactgggtga tgcaggttta caattacttt gcgaaggttt actggaccca 420

caatgtcgct tagaaaaact tcaattagaa tactgtagcc tgtcagcagc ctcttgtgaa 480

ccattagcgt ccgtcctgcg cgcaaaacca gattttaaag aacttaccgt gtctaataat 540

gatatcaacg aagctggcgt acgcgttctg tgtcaaggct taaaagattc accgtgtcaa 600

cttgaagctc tcaaactgga atcttgtggt gtgacttccg acaattgtcg tgacctgtgt 660

ggtattgtgg cttcaaaagc aagtctccgt gaattagccc tgggtagcaa taaattagga 720

gacgttggaa tggcagaact gtgtccgggc cttctgcatc cgtcatcgcg tttacgtacc 780

ctgtggatct gggaatgtgg tattacggca aaaggatgtg gcgacctctg tcgtgttctg 840

cgcgccaaag aaagcctgaa agaattgtct ttagcaggta atgaattagg tgatgaaggc 900

gctcgtctgt tatgcgaaac cttacttgaa ccgggttgtc agttagagtc tctgtgggtt 960

aaaagctgct cttttaccgc cgcctgttgt tcacattttt cctccgttct tgctcaaaac 1020

cgtttcttat tagaactcca aatcagtaac aaccgtcttg aagatgcggg cgttcgtgaa 1080

ttatgtcaag ggctgggcca accaggttcc gtcttacgtg tgttatggct ggctgattgc 1140

gatgtttccg attcatcgtg ctcttcactt gcggccaccc ttctcgctaa ccattcgctc 1200

cgtgaacttg atctttcgaa caactgtctt ggcgatgccg gtattctgca attagttgaa 1260

tcagtgcgtc aacctggctg cttactggaa cagctcgttc tctatgatat ttattggtcc 1320

gaagaaatgg aagaccgttt acaagccctg gaaaaagata aaccgtctct gcgtgttatt 1380

agctaagtcg acgcggccgc ataatgctta 1410

<210> 5
<211> 19
<212> DNA
<213> phage T7

<400> 5
taatacgact cactatagg 19

<210> 6
<211> 41
<212> DNA
<213> phage T7

<400> 6
aaccccttgg ggcctctaaa cgggtcttga ggggtttttt g 41

<210> 7
<211> 221
<212> DNA
<213> Escherichia coli

<400> 7
cgtagtttat tttgctttcc gagtcagctt tctgaccgcc tttcgtttta tctgttgttt 60

gtcggtgaac gctctcctga gtaggacaaa tccgccggga gcggatttga acgttgcgaa 120

gcaacggccc ggagggtggc gggcaggacg cccgccataa actgccaggc atcaaattaa 180

gcagaaggcc atcctgacgg atggcctttt tgcgtttcta c 221

<210> 8
<211> 25
<212> DNA
<213> Escherichia coli

<400> 8

ggaattgtga gcggataaca attcc                                                25

<210>  9
<211>  12
<212>  RNA
<213>  artificial

<220>
<223>  Core sequence of Sephadex aptamer

<400>  9
gaguaauuua cg                                                              12

<210>  10
<211>  20
<212>  DNA
<213>  artificial

<220>
<223>  primer for reverse transcription

<400>  10
gattgaccgc gcctcctatc                                                      20

<210>  11
<211>  20
<212>  DNA
<213>  artificial

<220>
<223>  forward  primer

<400>  11
agcaacagcg tgctacaagg                                                      20

<210>  12
<211>  20
<212>  DNA
<213>  artificial

<220>
<223>  reverse primer I

<400>  12
tgcccgcaca ttgaggaaac                                                      20

<210>  13
<211>  18
<212>  DNA
<213>  artificial

<220>
<223>  reverse primer  II

<400>  13
gagattgccc ccgtgtag                                                        18